# EUROPEAN PATENT APPLICATION

(11) **EP 1 852 104 A1**
(43) Date of publication of application: **07.11.2007**
(21) Application number: 06008058.7
(22) Date of filing: 19.04.2006
(51) Int. Cl.: A61K 8/49, A61Q 5/06, A61Q 5/10

(54) **Agents for colouring keratin fibres comprising zwitterionic azomethine dyes**

(71) Applicant: Wella Aktiengesellschaft, 64274 Darmstadt (DE)
(72) Inventor: Pasquier, Cécile, 1723 Marly (CH); Buclin, Véronique, 1638 Morlon (CH); Roulin, Anita, 1695 Villarlod (CH); Braun, Hans-Jürgen, 3182 Ueberstorf (CH)
(74) Representative: Bockhorst, Matthias

(57) **Abstract**

The present invention relates to agents for colouring keratin fibres which comprise at least one zwitterionic azomethine dye of the general formula (I), (II) or (III) 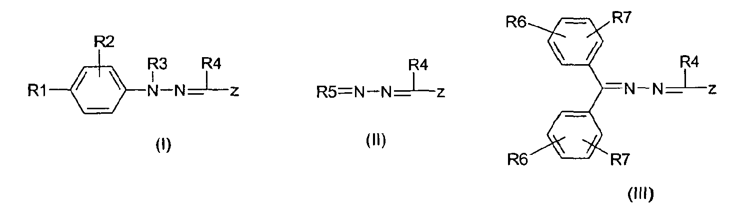 in which z is a heterocycle of the formula 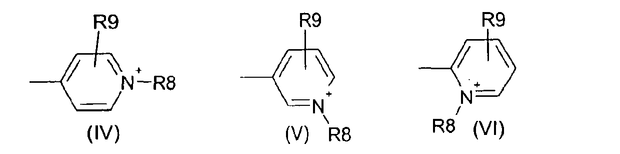 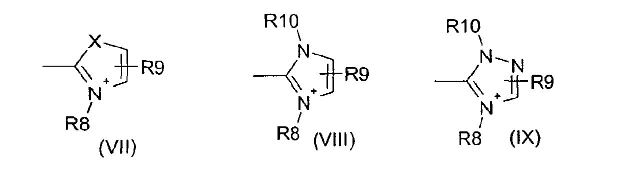 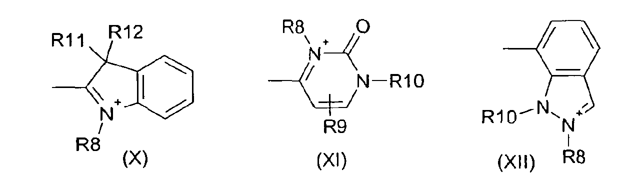 in which **R8** is an alkyl sulfonate radical of the formula (XIII);

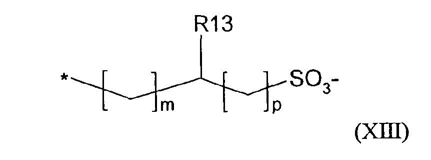

## Description

The present invention relates to agents for colouring keratin fibres, such as, for example, wool, and furs and, in particular, human hair, comprising zwitterionic azomethine dyes.

For the colour-changing treatment of keratin fibres use is usually made of two colouring methods. In the first method, the colouration is produced with so-called oxidative or permanent colourants using a mixture of various developer substances and coupler substances and an oxidizing agent. If required, in this method, so-called direct (nonoxidative) dyes can be added to round off the colouring result or to produce particular colour effects. The second method uses exclusively direct dyes, which are applied to the fibres in a suitable carrier mass. This method is easy to use, exceptionally gentle and is characterized by low damage to keratin fibres. The direct dyes used here are subject to a large number of requirements. For example, they have to be acceptable from a toxicological and dermatological point of view and allow the attainment of colourations in the desired intensity, which, inter alia, also requires adequate solubility in water. In addition, good lightfastness, acid fastness and rubbing fastness are required for the colourations achieved.
Compared with oxidative colourations, nonoxidative colourations, however, generally have lower durability and a poorer root to tip evenness of colour. In addition, direct colourants are generally not able to "lighten" the hair since many direct dyes do not withstand the oxidizing agents required for the lightening and/or the required pH of greater than or equal to 9.
WO 95/01772 A1 discloses specific azo dyes and azomethine dyes as well as their use for dyeing keratinic fibres. WO 97/20545 A1 discloses enlightning dyeing compositions for keratinic fibres which contain specific cationic dyes with a -N=N- or -CH=N- group.
US 3,573,287 discloses specific cationic azomethine compounds which are used for controlling infections of the urinary tract.

Surprisingly, it has now been found that certain zwitterionic azomethine or heteroaryl azine dyes intensely colour keratin fibres, and are oxidation-stable, and thus can also be used in oxidative colouring systems.

The present invention therefore provides
**(a)** an agent for the nonoxidative colouring of keratin fibres, in particular human hair;
**(b)** an agent for the simultaneous lightening and colouring of keratin fibres, in particular human hair, which, besides the dye of the formula (I), (II) or (III), comprises an oxidizing agent; and
**(c)** an oxidative colourant for keratin fibres, in particular human hair, based on at least one oxidation dye precursor;
where each of the agents **(a), (b)** and **(c)** is characterized by comprising at least one zwitterionic azomethine dye of the general formula (I), (II) or (III) 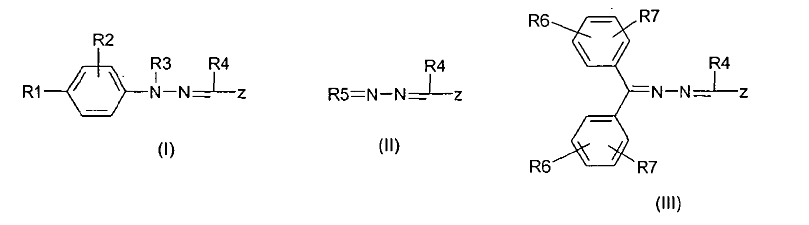 in which
**R1** and **R2** may be identical or different and indepedently of one another, are hydrogen, a halogen atom (F, Cl, Br, I), a saturated or unsaturated (C₁-C₁₂)-alkyl group, a (C₁-C₁₂)-alkyl group substituted by a halogen atom (F, Cl, Br, I), a hydroxyl group, a hydroxy-(C₁-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted C(O)O-phenyl group or an unsubstituted C(O)O-phenyl group, a substituted phenyl group or an unsubstituted phenyl group, a substituted naphthyl group or an unsubstituted naphthyl group, a substituted heteroaryl group or an unsubstituted heteroaryl group;
**R3** is an hydrogen, a saturated or unsaturated (C₁-C₁₂)-alkyl group, a hydroxy-(C₂-C₁₂)alkyl group, or forms with a carbon atom of the benzene ring a five or six-membered heterocycle which may be substituted with one or more (C₁-C₁₂)-alkyl group;
**R4** is hydrogen, a saturated or unsaturated (C₁-C₁₂)-alkyl group, a substituted phenyl group or an unsubstituted phenyl group;
**R5** is chosen from a group according to the general formula 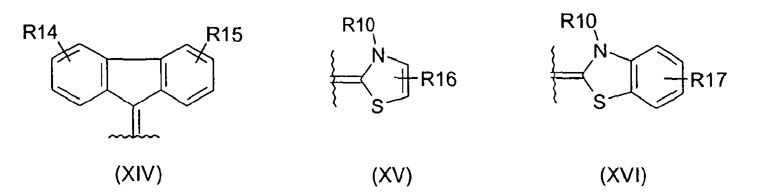 **R6** and **R7** may be identical or different and, independently of one another, are hydrogen, a halogen atom (F, Cl, Br, I), an amino group, a (C₁-C₁₂)-alkylamino group, a (C₁-C₁₂)-dialkylamino group, a C₁-C₆-alkylcyano group, a hydroxyl group, a nitro group, a saturated or unsaturated (C₁-C₁₂)-alkyl group, a hydroxy-(C₁-C₁₂)-alkyl group, a (C₁-C₁₂)-alkoxy group, a methoxy-(C₁-C₁₂)-alkyl group, a C(O)O-(C₁-C₁₂)-alkyl group;
**z** is a heterocycle of the formula (IV) to (XII) 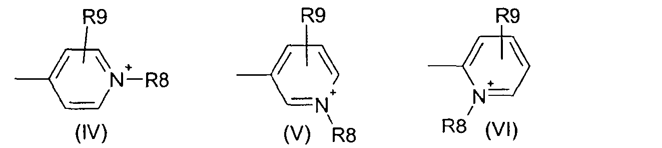 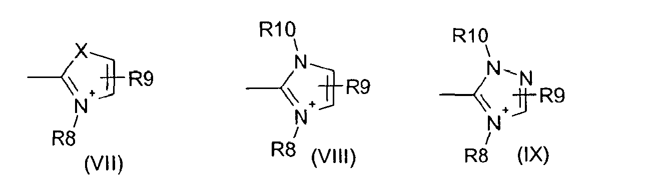 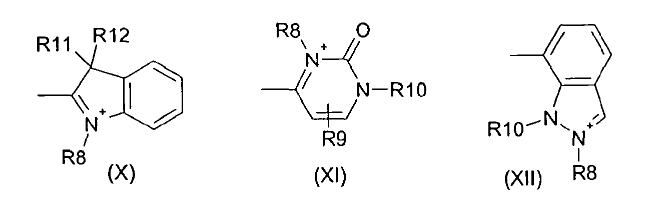 **X** is oxygen or sulfur;
**R8** is an alkyl sulfonate radical of the formula (XIII); 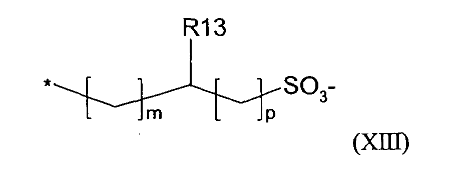 **R9** is hydrogen, a halogen atom (F, Cl, Br, I), a saturated or unsaturated (C₁-C₁₂)-alkyl group, a (C₁-C₁₂)-alkyl group substituted by a halogen atom (F, Cl, Br, I), a hydroxyl group, a hydroxy-(C₂-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted or unsubstituted C(O)O-phenyl group, a substituted or unsubstituted phenyl group, or a benzene ring condensed to the heteroaromatic ring of formula (IV) to (XII);
**R10** is a saturated or unsaturated (C₁-C₁₂)-alkyl group or a saturated or unsaturated hydroxy-(C₁-C₁₂)-alkyl group;
**R11** and **R12** may be identical or different and indepedently of one another, are a saturated or unsaturated (C₁-C₁₂)-alkyl group;
R13 is hydrogen or a hydroxyl group;
**R14, R15, R16** and **R17** may be identical or different and indepedently of one another, are hydrogen, a halogen atom (F, Cl, Br, I), a saturated or unsaturated (C₁-C₁₂)-alkyl group, a (C₁-C₁₂)-alkyl group substituted by a halogen atom (F, Cl, Br, I), a hydroxyl group, a hydroxy-(C₁-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted C(O)O-phenyl group or an unsubstituted C(O)O-phenyl group, a substituted phenyl group or an unsubstituted phenyl group,
**m** is equal to 0 to (n-1);
**p** is equal to 0 to (n-1) with m+p = (n-1) and
**n** is an integer from 1 to 6.

Among the above mentioned compounds of the formulas (I), (II) and (III), preference is given to those in which n is 2 or 3, and **R13** is hydrogen. The most preferred compounds are the compounds of formula (I) in which **n** is 2 or 3, and **R13** is hydrogen.

Examples of suitable compounds of the general formula (I), (II) or (III) that may be mentioned are:
4-(4- {[methyl(phenyl)hydrazono]methyl}-1-pyridiniumyl)-1-butanesulfonate,
4-(3-{[methyl(phenyl)hydrazono]methyl}-1-pyridiniumyl)-1-butanesulfonate,
4-(2-{[methyl(phenyl)hydrazono]methyl}-1-pyridiniumyl)-1-butanesulfonate,
4-(4-{[(4-methoxyphenyl)(methyl)hydrazono]methyl}-1-pyridiniumyl)-1-butanesulfonate,
4-(3-{[(4-methoxyphenyl)(methyl)hydrazono]methyl}-1-pyridiniumyl)-1-butanesulfonate,
4-(2-{[(4-methoxyphenyl)(methyl)hydrazono]methyl}-1-pyridiniumyl)-1-butanesulfonate,
4-(4-{[(2-methoxyphenyl)(methyl)hydrazono]methyl}-1-pyridiniumyl)-1-butanesulfonate,
4-(3-{[(2-methoxyphenyl)(methyl)hydrazono]methyl}-1-pyridiniumyl)-1-butanesulfonate,
4-(2-{[(2-methoxyphenyl)(methyl)hydrazono]methyl}-1-pyridiniumyl)-1-butanesulfonate,
4-(4-{[(4-hydroxyphenyl)(methyl)hydrazono]methyl}-1-pyridiniumyl)-1-butanesulfonate,
4-(3-{[(4-hydroxyphenyl)(methyl)hydrazono]methyl}-1-pyridiniumyl)-1-butanesulfonate,
4-(2-{[(4-hydroxyphenyl)(methyl)hydrazono]methyl}-1-pyridiniumyl)-1-butanesulfonate,
4-(4-{[methyl(4-methylphenyl)hydrazono]methyl}-1-pyridiniumyl)-1-butanesulfonate,
4-(3-{[methyl(4-methylphenyl)hydrazono]methyl}-1-pyridiniumyl)-1-butanesulfonate,
4-(2-{[methyl(4-methylphenyl)hydrazono]methyl}-1-pyridiniumyl)-1-butanesulfonate,
4-(4-{[(4-chlorophenyl)(methyl)hydrazono]methyl}-1-pyridiniumyl)-1-butanesulfonate,
4-(3-{[(4-chlorophenyl)(methyl)hydrazono]methyl}-1-pyridiniumyl)-1-butanesulfonate,
4-(2-{[(4-chlorophenyl)(methyl)hydrazono]methyl}-1-pyridiniumyl)-1-butanesulfonate,
4-(4-{[methyl(4-nitrophenyl)hydrazono]methyl}-1-pyridiniumyl)-1-butanesulfonate,
4-(3-{[methyl(4-nitrophenyl)hydrazono]methyl}-1-pyridiniumyl)-1-butanesulfonate,
4-(2-{[methyl(4-nitrophenyl)hydrazono]methyl}-1-pyridiniumyl)-1-butanesulfonate,
4-{4-[(phenylhydrazono)methyl]-1-pyridiniumyl}-1-butanesulfonate,
4-{3-[(phenylhydrazono)methyl]-1-pyridiniumyl}-1-butanesulfonate,
4-{2-[(phenylhydrazono)methyl]-1-pyridiniumyl}-1-butanesulfonate,
4-(4-{[(4-methoxyphenyl)hydrazono]methyl}-1-pyridiniumyl)-1-butanesulfonate,
4-(3-{[(4-methoxyphenyl)hydrazono]methyl}-1-pyridiniumyl)-1-butanesulfonate,
4-(2-{[(4-methoxyphenyl)hydrazono]methyl}-1-pyridiniumyl)-1-butanesulfonate,
4-{4-[*N*-methyl-*N*-phenylethanehydrazonoyl]-1-pyridiniumyl}-1-butanesulfonate,
4-{3-[*N*-methyl-*N*-phenylethanehydrazonoyl]-1-pyridiniumyl}-1-butanesulfonate,
4-{2-[*N*-methyl-*N*-phenylethanehydrazonoyl]-1-pyridiniumyl}-1-butanesulfonate,
4-{4-[*N*-phenylethanehydrazonoyl]-1-pyridiniumyl}-1-butanesulfonate,
4-{3-[*N*-phenylethanehydrazonoyl]-1-pyridiniumyl}-1-butanesulfonate,
4-{2-[*N*-phenylethanehydrazonoyl]-1-pyridiniumyl}-1-butanesulfonate,
4-(2-{[methyl(phenyl)hydrazono]methyl}-1,3-thiazol-3-ium-3-yl)-1-butanesulfonate,
4-(1-methyl-2- {[methyl(phenyl)hydrazono]methyl}-1H-imidazol-3-ium-3-yl)-1-butanesulfonate,
4-(2-{[methyl(phenyl)bydrazono]methyl}-1,3-oxazol-3-ium-3-yl)-1-butanesulfonate,
4-(4-methyl-5-{[methyl(phenyl)hydrazono]methyl}-4*H*-1,2,4-triazol-1-ium-1-yl)-1-butanesulfonate,
4-(4-{[methyl(phenyl)hydrazono]methyl}-1-quinoliniumyl)-1-butanesulfonate,
4-(2-{[methyl(phenyl)hydrazono]methyl}-1-quinoliniumyl)-1-butanesulfonate,
4-{4-[(2,3-dihydro-1*H*-indol-1-ylimino)methyl]-1-pyridiniumyl}-1-butanesulfonate,
4-(4-{[(2,2,3,3-tetramethyl-2,3-dihydro-1*H*-indol-1-yl)imino]methyl}-1-pyridiniumyl)-1-butanesulfonate,
4-(3,3-dimethyl-2-{[methyl(phenyl)hydrazono]methyl}-3*H*-indolium-1-yl)-1-butanesulfonate,
4-(2-{[(4-methoxyphenyl)(methyl)hydrazono]methyl}-3,3-dimethyl-3*H*-indolium-1-yl)-1-butanesulfonate,
4-(3-methyl-6-{[methyl(phenyl)hydrazono]methyl}-2-oxo-2,3-dihydropyrimidin-1-ium-1-yl)-1-butanesulfonate,
4-(1-methyl-7-{[methyl(phenyl)hydrazono]methyl}-1H-indazol-2-ium-2-yl)-1-butanesulfonate,
3-(4-{[methyl(phenyl)hydrazono]methyl}-1-pyridiniumyl)-1-propanesulfonate,
3-(3-{[methyl(phenyl)hydrazono]methyl}-1-pyridiniumyl)-1-propanesulfonate,
3-(2-{[methyl(phenyl)hydrazono]methyl}-1-pyridiniumyl)-1-propanesulfonate,
3-(4-{[(4-methoxyphenyl)(methyl)hydrazono]methyl}-1-pyridiniumyl)-1-propanesulfonate,
3-(3-{[(4-methoxyphenyl)(methyl)hydrazono]methyl}-1-pyridiniumyl)-1-propanesulfonate,
3-(2-{[(4-methoxyphenyl)(methyl)hydrazono]methyl}-1-pyridiniumyl)-1-propanesulfonate,
3-(4-{[(2-methoxyphenyl)(methyl)hydrazono]methyl}-1-pyridiniumyl)-1-propanesulfonate,
3-(3-{[(2-methoxyphenyl)(methyl)hydrazono]methyl}-1-pyridiniumyl)-1-propanesulfonate,
3-(2-{[(2-methoxyphenyl)(methyl)hydrazono]methyl}-1-pyridiniumyl)-1-propanesulfonate,
3-(4-{[(4-hydroxyphenyl)(methyl)hydrazono]methyl}-1-pyridiniumyl)-1-propanesulfonate,
3-(3-{[(4-hydroxyphenyl)(methyl)hydrazono]methyl}-1-pyridiniumyl)-1-propanesulfonate,
3-(2-{[(4-hydroxyphenyl)(methyl)hydrazono]methyl}-1-pyridiniumyl)-1-propanesulfonate,
3-(4-{[methyl(4-methylphenyl)hydrazono]methyl}-1-pyridiniumyl)-1-propanesulfonate,
3-(3-{[methyl(4-methylphenyl)hydrazono]methyl}-1-pyridiniumyl)-1-propanesulfonate,
3-(2-{[methyl(4-methylphenyl)hydrazono]methyl}-1-pyridiniumyl)-1-propanesulfonate,
3-(4-{[(4-chlorophenyl)(methyl)hydrazono]methyl}-1-pyridiniumyl)-1-propanesulfonate,
3-(3-{[(4-chlorophenyl)(methyl)hydrazono]methyl}-1-pyridiniumyl)-1-propanesulfonate,
3-(2-{[(4-chlorophenyl)(methyl)hydrazono]methyl}-1-pyridiniumyl)-1-propanesulfonate,
3-(4-{[methyl(4-nitrophenyl)hydrazono]methyl}-1-pyridiniumyl)-1-propanesulfonate,
3-(3-{[methyl(4-nitrophenyl)hydrazono]methyl}-1-pyridiniumyl)-1-propanesulfonate,
3-(2-{[methyl(4-nitrophenyl)hydrazono]methyl}-1-pyridiniumyl)-1-propanesulfonate,
3-{4-[(phenylhydrazono)methyl]-1-pyridiniumyl}-1-propanesulfonate,
3-{3-[(phenylhydrazono)methyl]-1-pyridiniumyl}-1-propanesulfonate,
3-{2-[(phenylhydrazono)methyl]-1-pyridiniumyl}-1-propanesulfonate,
3-(4-{[(4-methoxyphenyl)hydrazono]methyl}-1-pyridiniumyl)-1-propanesulfonate,
3-(3-{[(4-methoxyphenyl)hydrazono]methyl}-1-pyridiniumyl)-1-propanesulfonate,
3-(2-{[(4-methoxyphenyl)hydrazono]methyl}-1-pyridiniumyl)-1-propanesulfonate,
3-{4-[*N*-methyl-*N*-phenylethanehydrazonoyl]-1-pyridiniumyl}-1-propanesulfonate,
3-{3-[*N*-methyl-*N*-phenylethanehydrazonoyl]-1-pyridiniumyl}-1-propanesulfonate,
3-{2-[*N*-methyl-*N*-phenylethanehydrazonoyl]-1-pyridiniumyl}-1-propanesulfonate,
3-{4-[*N*-phenylethanehydrazonoyl]-1-pyridiniumyl}-1-propanesulfonate,
3-{3-[*N*-phenylethanehydrazonoyl]-1-pyridiniumyl}-1-propanesulfonate,
3-{2-[*N*-phenylethanehydrazonoyl]-1-pyridiniumyl}-1- propanesulfonate,
3-(2-{[methyl(phenyl)hydrazono]methyl}-1,3-thiazol-3-ium-3-yl)-1-propanesulfonate,
3-(1-methyl-2-{[methyl(phenyl)hydrazono]methyl}-1H-imidazol-3-ium-3-yl)-1-propanesulfonate,
3-(2-{[methyl(phenyl)hydrazono]methyl}-1,3-oxazol-3-ium-3-yl)-1-propanesulfonate,
3-(4-methyl-5-{[methyl(phenyl)hydrazono]methyl}-4*H*-1,2,4-triazol-1-ium-1-yl)-1-propanesulfonate,
3-(4-{[methyl(phenyl)hydrazono]methyl}-1-quinoliniumyl)-1-propanesulfonate,
3-(2-{[methyl(phenyl)hydrazono]methyl}-1-quinoliniumyl)-1-propanesulfonate,
3-{4-[(2,3-dihydro-1*H*-indol-1-ylimino)methyl]-1-pyridiniumyl}-1-propanesulfonate
3-(4-{[(2,2,3,3-tetramethyl-2,3-dihydro-1*H*-indol-1-yl)imino]methyl}-1-pyridiniumyl)-1-propanesulfonate,
3-(3,3-dimethyl-2-{[methyl(phenyl)hydrazono]methyl}-3*H*-indolium-1-yl)-1-propanesulfonate,
3-(2-{[(4-methoxyphenyl)(methyl)hydrazono]methyl}-3,3-dimethyl-3*H*-indolium-1-yl)-1-propanesulfonate,
3-(3-methyl-6-{[methyl(phenyl)hydrazono]methyl}-2-oxo-2,3-dihydropyrimidin-1-ium-1-yl)-1-propanesulfonate,
3-(1-methyl-7-{[methyl(phenyl)hydrazono]methyl}-1*H*-indazol-2-ium-2-yl)-1-propanesulfonate,
4-(4-{[(3-methyl-1,3-benzothiazol-2(3H)-ylidene)hydrazono]methyl}-1-pyridiniumyl)-1-butanesulfonate,
4-(3-{[(3-methyl-1,3-benzothiazol-2(3H)-ylidene)hydrazono]methyl}-1-pyridiniumyl)-1-butanesulfonate,
4-(4-{[(3,4-dimethyl-1,3-thiazol-2(3H)-ylidene)hydrazono]methyl}-1-pyridiniumyl)-1-butanesulfonate,
4-(3-{[(3,4-dimethyl-1,3-thiazol-2(3H)-ylidene)hydrazono]methyl}-1-pyridiniumyl)-1-butanesulfonate,
4-(4-{[(3,4,5-trimethyl-1,3-thiazol-2(3H)-ylidene)hydrazono]methyl}-1-pyridiniumyl)-1-butanesulfonate,
4-(3-{[(3,4,5-trimethyl-1,3-thiazol-2(3H)-ylidene)hydrazono]methyl}-1-pyridiniumyl)-1-butanesulfonate,
4-(1-methyl-2-{[2-(3-methyl-1,3-benzothiazol-2(3*H*)-ylidene)hydrazono]methyl}-1*H-*imidazol-3-ium-3-yl)-1-butanesulfonate,
4-(2-{[2-(3,4-dimethyl-1,3-thiazol-2(3*H*)-ylidene)hydrazono]methyl}-1-methyl-1*H-*imidazol-3-ium-3-yl)-1-butanesulfonate,
4-(2-{[2-(3,4,5-trimethyl-1,3-thiazol-2(3*H*)-ylidene)hydrazono]methyl}-1-methyl-1*H-*imidazol-3-ium-3-yl)-1-butanesulfonate,
4-{4-[({bis[4-(dimethylamino)phenyl]methylene}hydrazono)methyl]-1-pyridiniumyl}-1-butanesulfonate,
4-{3-[({bis[4-(dimethylamino)phenyl]methylene)hydrazono)methyl]-1-pyridiniumyl}-1-butanesulfonate,
4-{4-[(9*H*-fluoren-9-ylidenehydrazono)methyl]-1-pyridiniumyl}-1-butanesulfonate,
4-{4-[({bis[4-(dimethylamino)phenyl]methylene}hydrazono)methyl]-1-quinoliniumyl}-1-butanesulfonate,
3-(4-{[(3-methyl-1,3-benzothiazol-2(3H)-ylidene)hydrazono]methyl}-1-pyridiniumyl)-1-propanesulfonate,
3-(3-{[(3-methyl-1,3-benzothiazol-2(3H)-ylidene)hydrazono]methyl}-1-pyridiniumyl)-1-propanesulfonate,
3-(4-{[(3,4-dimethyl-1,3-thiazol-2(3H)-ylidene)hydrazono]methyl}-1-pyridiniumyl)-1-propanesulfonate,
3-(3-{[(3,4-dimethyl-1,3-thiazol-2(3H)-ylidene)hydrazono]methyl}-1-pyridiniumyl)-1-propanesulfonate,
3-(4-{[(3,4,5-trimethyl-1,3-thiazol-2(3H)-ylidene)hydrazono]methyl}-1-pyridiniumyl)-1-propanesulfonate,
3-(3-{[(3,4,5-trimethyl-1,3-thiazol-2(3H)-ylidene)hydrazono]methyl}-1-pyridiniumyl)-1-propanesulfonate,
3-(1-methyl-2-{[2-(3-methyl-1,3-benzothiazol-2(3*H*)-ylidene)hydrazono]methyl}-1*H-*imidazol-3-ium-3-yl)-1-propanesulfonate,
3-(2-{[2-(3,4-dimethyl-1,3-thiazol-2(3*H*)-ylidene)hydrazono]methyl}-1-methyl-1*H-*imidazol-3-ium-3-yl)-1-propanesulfonate,
3-(2-{[2-(3,4,5-trimethyl-1,3-thiazol-2(3*H*)-ylidene)hydrazono]methyl}-1-methyl-1*H-*imidazol-3-ium-3-yl)-1-propanesulfonate,
3-{4-[({bis[4-(dimethylamino)phenyl]methylene}hydrazono)methyl]-1-pyridiniumyl}-1-propanesulfonate,
3-{3-[({bis[4-(dimethylamino)phenyl]methylene}hydrazono)methyl]-1-pyridiniumyl}-1-propanesulfonate,
3-{4-[(9*H*-fluoren-9-ylidenehydrazono)methyl]-1-pyridiniumyl}-1-propanesulfonate and
3-{4-[({bis[4-(dimethylamino)phenyl]methylene}hydrazono)methyl]-1-quinoliniumyl}-1-propanesulfonate.

The dyes of the formula (I), (II) and (III) are present in the colourant according to the invention preferably in a total amount of from 0.01 to 10% by weight, in particular 0.1 to 8% by weight.

To extend the colour pallet, the colourant (a) according to the invention besides the dyes of the formula (I), may additionally also comprise further known direct synthetic dyes from the group consisting of nitro dyes, azo dyes, anthraquinone dyes, triphenylmethane dyes and basic or acidic dyes, and natural direct dyes, alone or in a mixture with one another, for example 1,4-bis[(2-hydroxyethyl)amino]-2-nitrobenzene, 1-(2-hydroxyethyl)amino-2-nitro-4-[di(2-hydroxyethyl)amino]benzene, (HC Blue No. 2), 1-amino-3-methyl-4-[(2-hydroxyethyl)amino]-6-nitrobenzene, (HC Violet No. 1), 4-[ethyl-(2-hydroxyethyl)amino]-1-[(2-hydroxyethyl)amino]-2-nitrobenzene hydrochloride (HC Blue No. 12), 4-[di(2-hydroxyethyl)amino]-1-[(2-methoxyethyl)amino]-2-nitrobenzene (HC Blue No. 11), 1-[(2,3-dihydroxypropyl)amino]-4-[methyl-(2-hydroxyethyl)amino]-2-nitrobenzene (HC Blue No. 10), 1-[(2,3-dihydroxypropyl)amino]-4-[ethyl-(2-hydroxyethyl)amino]-2-nitrobenzene hydrochloride (HC Blue No. 9), 1-(3-hydroxypropylamino)-4-[di(2-hydroxyethyl)amino]-2-nitrobenzene, (HC Violet No. 2), 1-methylamino-4-[methyl-(2,3-dihydroxypropyl)amino]-2-nitrobenzene (HC Blue No. 6), 2-((4-amino-2-nitrophenyl)amino)-5-dimethylaminobenzoic acid (HC Blue No. 13), 1-(2-aminoethylamino)-4-[di(2-hydroxyethyl)amino]-2-nitrobenzene, 4-(di(2-hydroxyethyl)amino)-2-nitro-1-phenylaminobenzene,1-amino-4-[(2-hydroxyethyl)amino]-2-nitrobenzene (HC Red No. 7), 2-amino-4,6-dinitrophenol, 1,4-diamino-2-nitrobenzene (CI76070), 4-amino-2-nitrodiphenylamine (HC Red No. 1), 1-amino-4-[di(2-hydroxyethyl)amino]-2-nitrobenzene hydrochloride (HC Red No. 13), 1-amino-5-chloro-4-[(2-hydroxyethyl)amino]-2-nitrobenzene, 4-amino-1-[(2-hydroxyethyl)amino]-2-nitrobenzene (HC Red No. 3), 4-((2-hydroxyethyl)methylamino)-1-(methylamino)-2-nitrobenzene, 1-amino-4-((2,3-dihydroxypropyl)amino)-5-methyl-2-nitrobenzene, 1-amino-4-(methylamino)-2-nitrobenzene,4-amino-2-nitro-1-((prop-2-en-1-yl)amino)benzene, 4-amino-3-nitrophenol, 4-[(2-hydroxyethyl)amino]-3-nitrophenol, 4-[(2-nitrophenyl)amino]phenol (HC Orange No. 1), 1-[(2-aminoethyl)amino]-4-(2-hydroxyethoxy)-2-nitrobenzene (HC Orange No. 2), 4-(2,3-dihydroxypropoxy)-1-[(2-hydroxyethyl)amino]-2-nitrobenzene, (HC Orange No. 3), 1-amino-5-chloro-4-[(2,3-dihydroxypropyl)amino]-2-nitrobenzene (HC Red No. 10), 5-chloro-1,4-[di(2,3-dihydroxypropyl)amino]-2-nitrobenzene (HC Red No. 11), 2-[(2-hydroxyethyl)amino]-4,6-dinitrophenol, 4-ethylamino-3-nitrobenzoic acid, 2-[(4-amino-2-nitrophenyl)amino]benzoic acid, 2-chloro-6-ethylamino-4-nitrophenol, 2-amino-6-chloro-4-nitrophenol, 4-[(3-hydroxypropyl)amino]-3-nitrophenol, 2,5-diamino-6-nitropyridine, 6-amino-3-((2-hydroxyethyl)-amino)-2-nitropyridine, 3-amino-6-((2-hydroxyethyl)amino)-2-nitropyridine, 3-amino-6-(ethylamino)-2-nitropyridine, 3-((2-hydroxyethyl)amino)-6-(methylamino)-2-nitropyridine, 3-amino-6-(methylamino)-2-nitropyridine, 6-(ethylamino)-3-((2-hydroxyethyl)amino)-2-nitropyridine, 1,2,3,4-tetrahydro-6-nitroquinoxaline, 7-amino-3,4-dihydro-6-nitro-2H-1,4-benzoxazine (HC Red No. 14), 1,2-diamino-4-nitrobenzene (CI76020), 1-amino-2-[(2-hydroxyethyl)amino]-5-nitrobenzene (HC Yellow No. 5), 1-(2-hydroxyethoxy)-2-[(2-hydroxyethyl)amino]-5-nitrobenzene, (HC Yellow No. 4), 1-[(2-hydroxyethyl)amino]-2-nitrobenzene (HC Yellow No. 2), 2-(di(2-hydroxyethyl)amino)-5-nitrophenol, 2-[(2-hydroxyethyl)amino]-1-methoxy-5-nitrobenzene, 2-amino-3-nitrophenol, 1-amino-2-methyl-6-nitrobenzene, 1-(2-hydroxyethoxy)-3-methylamino-4-nitrobenzene, 2,3-(dihydroxypropoxy)-3-methylamino-4-nitrobenzene, 2-[(2-hydroxyethyl)amino]-5-nitrophenol (HC Yellow No. 11), 3-[(2-aminoethyl)amino]-1-methoxy-4-nitrobenzene hydrochloride, (HC Yellow No.9), 1-[(2-ureidoethyl)amino]-4-nitrobenzene, 4-[(2,3-dihydroxypropyl)amino]-3-nitro-1-trifluoromethylbenzene, (HC Yellow No. 6), 1-chloro-2,4-bis[(2-hydroxyethyl)amino]-5-nitrobenzene (HC Yellow No. 10), 1-amino-4-((2-aminoethyl)amino)-5-methyl-2-nitrobenzene, 4-[(2-hydroxyethyl)amino]-3-nitro-1-methylbenzene, 1-chloro-4-[(2-hydroxyethyl)amino]-3-nitrobenzene (HC Yellow No. 12), 4-[(2-hydroxyethyl)amino]-3-nitro-1-trifluoromethylbenzene, (HC Yellow No. 13), 4-[(2-hydroxyethyl)amino]-3-nitrobenzonitrile (HC Yellow No. 14), 4-[(2-hydroxyethyl)amino]-3-nitrobenzamide (HC Yellow No. 15), 3-((2-hydroxyethyl)amino)-4-methyl-1-nitrobenzene, 4-chloro-3-((2-hydroxyethyl)amino)-1-nitrobenzene, 2,4-dinitro-1-hydroxynaphthalene, 1,4-di[(2,3-dihydroxypropyl)amino]-9,10-anthraquinone, 1,4-di[(2-hydroxyethyl)amino]-9,10-anthraquinone (CI61545, Disperse Blue 23), 1-[(2-hydroxyethyl)amino]-4-methylamino-9,10-anthraquinone (CI61505, Disperse Blue No. 3), 2-[(2-aminoethyl)amino]-9,10-anthraquinone (HC Orange No. 5), 1-amino-4-hydroxy-9,10-anthraquinone (CI60710, Disperse Red 15), 1-hydroxy-4-[(4-methyl-2-sulfophenyl)amino]-9,10-anthraquinone, 7-beta-D-glucopyranosyl-9,10-dihydro-1-methyl-9,10-dioxo-3,5,6,8-tetrahydroxy-2-anthracenecarboxylic acid (CI75470, Natural Red 4), 1-[(3-aminopropyl)amino]-4-methylamino-9,10-anthraquinone (HC Blue No. 8), 1-[(3-aminopropyl)amino]-9,10-anthraquinone (HC Red No. 8), 1,4-diamino-2-methoxy-9,10-anthraquinone (CI62015, Disperse Red No. 11, Solvent Violet No. 26), 1,4-dihydroxy-5,8-bis[(2-hydroxyethyl)amino]-9,10-anthraquinone (CI62500, Disperse Blue No. 7, Solvent Blue No. 69), 1,4-diamino-9,10-anthraquinone (CI61100, Disperse Violet No. 1), 1-amino-4-(methylamino)-9,10-anthraquinone (CI61105, Disperse Violet No. 4, Solvent Violet No. 12), 2-hydroxy-3-methoxy-1,4-naphthoquinone, 2,5-dihydroxy-1,4-naphthoquinone, 2-hydroxy-3-methyl-1,4-naphthoquinone, N-(6-((3-chloro-4-(methylamino)phenyl)imino)-4-methyl-3-oxo-1,4-cyclohexadien-1-yl)urea (HC Red No. 9), 2-((4-(di(2-hydroxyethyl)amino)phenyl)amino)-5-((2-hydroxyethyl)amino)-2,5-cyclohexadiene-1,4-dione (HC Green No. 1), 5-hydroxy-1,4-naphthoquinone (CI75500, Natural Brown No. 7), 2-hydroxy-1,4-naphthoquinone (CI75480, Natural Orange No. 6), 1,2-dihydro-2-(1,3-dihydro-3-oxo-2H-indol-2-ylidene)-3H-indol-3-one (CI73000), 1,3-bis(dicyanomethylene)indane, 9-(dimethylamino)benzo[a]phenoxazin-7-ium chloride (CI51175; Basic Blue No. 6), di[4-(diethylamino)phenyl][4-(ethylamino)naphthyl]-carbenium chloride (CI42595; Basic Blue No. 7), di(4-(dimethylamino)phenyl)(4-(methylphenylamino)naphthalen-1-yl)carbenium chloride (CI42563; Basic Blue No. 8), 3,7-di(dimethylamino)phenothiazin-5-ium chloride (CI52015; Basic Blue No. 9), di[4-(dimethylamino)phenyl][4-(phenylamino)naphthyl]carbenium chloride (CI44045; Basic Blue No. 26), 2-[(4-(ethyl(2-hydroxyethyl)amino)-phenyl)azo]-6-methoxy-3-methylbenzothiazolium methylsulfate (CI11154; Basic Blue No. 41), Basic Blue No. 77, 8-amino-2-bromo-5-hydroxy-4-imino-6-[(3-(trimethylammonio)phenyl)amino]-1 (4H)-naphthalenone chloride (CI56059; Basic Blue No. 99), bis[4-(dimethylamino)phenyl][4-(methylamino)phenyl]carbenium chloride (CI42535; Basic Violet No. 1), tri(4-amino-3-methylphenyl)carbenium chloride (CI42520; Basic Violet No. 2), tris[4-(dimethylamino)phenyl]carbenium chloride (CI42555; Basic Violet No. 3), 2-[3,6-(diethylamino)dibenzopyranium-9-yl]benzoyl chloride (CI45170; Basic Violet No. 10), di(4-aminophenyl)(4-amino-3-methylphenyl)carbenium chloride (CI42510; Basic Violet No. 14),1,3-bis[(2,4-diamino-5-methylphenyl)azo]-3-methylbenzene (CI21010; Basic Brown No. 4), 1-[(4-aminophenyl)azo]-7-(trimethylammonio)-2-naphthol chloride (CI12250; Basic Brown No. 16), 3-[(4-amino-2,5-dimethoxyphenyl)azo]-N,N,N-trimethylbenzenaminium chloride (CI112605, Basic Orange No. 69), 1-[(4-amino-2-nitrophenyl)azo]-7-(trimethylammonio)-2-naphthol chloride (Basic Brown No. 17), 1-[(4-amino-3-nitrophenyl)azo]-7-(trimethylammonio)-2-naphthol chloride (CI12251; Basic Brown No. 17), 2-((4-aminophenyl)azo)-1,3-dimethyl-1H-imidazol-3-ium chloride (Basic Orange No. 31), 3,7-diamino-2,8-dimethyl-5-phenylphenazinium chloride (CI50240; Basic Red No. 2),1,4-dimethyl-5-[(4-(dimethylamino)phenyl)azo]-1,2,4-triazolium chloride (CI11055; Basic Red No. 22), 1,3-dimethyl-2-((4-dimethylamino)phenyl)-azo-1H-imidazol-3-ium chloride (Basic Red No. 51), 2-hydroxy-1-[(2-methoxyphenyl)azo]-7-(trimethylammonio)naphthalene chloride (CI12245; Basic Red No. 76), 2-[2-((2,4-dimethoxyphenyl)amino)ethenyl]-1,3,3-trimethyl-3H-indol-1-ium chloride (CI48055; Basic Yellow No. 11), 3-methyl-1-phenyl-4-[(3-(trimethylammonio)phenyl)azo]pyrazol-5-one chloride (CI12719; Basic Yellow No. 57), di[4-(dimethylamino)phenyl]-iminomethane hydrochloride (CI41000; Basic Yellow No. 2), 1-methyl-4-((methylphenylhydrazono)methyl)pyridinium methylsulfate (Basic Yellow No. 87), bis[4-(diethylamino)phenyl]phenylcarbenium hydrogensulfate (1:1) (CI42040; Basic Green No. 1), di(4-(dimethylamino)phenyl)-phenylmethanol (CI42000; Basic Green No. 4), 1-(2-morpholinium-propylamino)-4-hydroxy-9,10-anthraquinone methylsulfate, 1-[(3-(dimethylpropylaminium)propyl)amino]-4-(methylamino)-9,10-anthraquinone chloride, 1-[di(2-hydroxyethyl)amino]-3-methyl-4-[(4-nitrophenyl)azo]benzene (CI11210, Disperse Red No. 17), 1-[di(2-hydroxyethyl)amino]-4-[(4-nitrophenyl)azo]benzene (Disperse Black No. 9), 4-[(4-aminophenyl)azo]-1-[di(2-hydroxyethyl)amino]-3-methylbenzene, (HC Yellow No. 7), 2,6-diamino-3-[(pyridin-3-yl)azo]pyridine, 2-((4-(acetylamino)phenyl)azo)-4-methylphenol (CI11855; Disperse Yellow No. 3), 2-((4-(ethyl(2-hydroxyethyl)amino)-2-methylphenyl)azo)-5-nitro-1,3-thiazole (CI111935; Disperse Blue No. 106), 6-hydroxy-5-[(4-sulfophenyl)azo]-2-naphthalenesulfonic acid disodium salt (CI15985; Food Yellow No. 3; FD&C Yellow No. 6), 2,4-dinitro-1-naphthol-7-sulfonic acid disodium salt (CI10316; Acid Yellow No. 1; Food Yellow No. 1), 2-(indane-1,3-dion-2-yl)quinoline-x,x-sulfonic acid (mixture of mono- and disulfonic acid) (CI47005; D&C Yellow No. 10; Food Yellow No. 13, Acid Yellow No. 3), 5-hydroxy-1-(4-sulfophenyl)-4-[(4-sulfophenyl)azo]pyrazole-3-carboxylic acid trisodium salt (CI19140; Food Yellow No. 4; Acid Yellow No. 23), 9-(2-carboxyphenyl)-6-hydroxy-3H-xanthen-3-one (CI45350; Acid Yellow No. 73; D&C Yellow No. 8), 4-((4-amino-3-sulfophenyl)azo)benzenesulfonic acid disodium salt (CI13015, Acid Yellow No. 9), 5-[(2,4-dinitrophenyl)amino]-2-phenylaminobenzenesulfonic acid sodium salt (CI10385; Acid Orange No. 3), 4-[(2,4-dihydroxyphenyl)azo]benzenesulfonic acid monosodium salt (CI14270; Acid Orange No. 6), 4-[(2-hydroxynaphth-1-yl)azo]benzenesulfonic acid sodium salt (CI15510; Acid Orange No. 7), 4-((2-hydroxynaphthalen-1-yl)azo)-3-methylbenzenesulfonic acid sodium salt (CI15575; Acid Orange No. 8), 4-[(2,4-dihydroxy-3-[(2,4-dimethylphenyl)azo]phenyl)azo]-benzenesulfonic acid sodium salt (CI20170; Acid Orange No. 24),3',6'-dihydroxy-4',5'-diiodospiro(isobenzofuran-1(3H)-9'-(9H)xanthen)-3-one (CI45425, D&C Orange No. 10), 4-hydroxy-3-[(4-sulfonaphth-1-yl)azo]-1-naphthalenesulfonic acid disodium salt (CI14720; Acid Red No. 14), 4-hydroxy-3-[(2-methoxyphenyl)azo]-1-naphthalenesulfonic acid monosodium salt (CI14710; Acid Red No. 4), 6-hydroxy-5-[(4-sulfonaphth-1-yl)azo]-2,4-naphthalenedisulfonic acid trisodium salt (CI16255; Ponceau 4R; Acid Red No. 18), 3-hydroxy-4-[(4-sulfonaphth-1-yl)azo]-2,7-naphthalenedisulfonic acid trisodium salt (CI16185; Acid Red No. 27), 8-amino-1-hydroxy-2-(phenylazo)-3,6-naphthalenedisulfonic acid disodium salt (CI17200; Acid Red No. 33), 5-(acetylamino)-4-hydroxy-3-[(2-methylphenyl)azo]-2,7-naphthalenedisulfonic acid disodium salt (CI18065; Acid Red No. 35), 2-(3-hydroxy-2,4,5,7-tetraiododibenzopyran-6-on-9-yl)benzoic acid disodium salt (CI45430; Acid Red No. 51), N-[6-(diethylamino)-9-(2,4-disulfophenyl)-3H-xanthen-3-ylidene]-N-ethylethaneammonium hydroxide, internal salt, sodium salt (CI45100; Acid Red No. 52), 8-[(4-(phenylazo)phenyl)azo]-7-naphthol-1,3-disulfonic acid disodium salt (CI27290; Acid Red No. 73), 2',4',5',7'-tetrabromo-3',6'-dihydroxyspiro[isobenzofuran-1(3H),9'-[9H]xanthen]-3-one disodium salt (CI45380; Acid Red No. 87), 2',4',5',7'-tetrabromo-4,5,6,7-tetrachloro-3',6'-dihydroxyspiro[isobenzofuran-1(3H),9'-[9H]xanthen]-3-one disodium salt (CI45410; Acid Red No. 92), 3',6'-dihydroxy-4',5'-diiodospiro[isobenzofuran-1(3H),9'-(9H)-xanthen]-3-one disodium salt (CI45425; Acid Red No. 95), 2-hydroxy-3-((2-hydroxynaphth-1-yl)azo)-5-nitrobenzenesulfonic acid monosodium salt (CI15685; Acid Red No. 184), (2-sulfophenyl)di[4-(ethyl((4-sulfophenyl)methyl)amino)phenyl]carbenium disodium salt, betaine (CI42090; Acid Blue No. 9; FD&C Blue No. 1), 3-hydroxy-4-((4-methyl-2-sulfophenyl)azo)-2-naphthalenecarboxylic acid disodium salt (CI15850; D&C Red No. 6), 6-hydroxy-5-((2-methoxy-5-methyl-4-sulfophenyl)azo)-2-naphthalenesulfonic acid disodium salt (CI16035; FD&C Red 40), 1,4-bis[(2-sulfo-4-methylphenyl)amino]-9,10-anthraquinone disodium salt (CI61570; Acid Green No. 25), bis[4-(dimethylamino)phenyl](3,7-disulfo-2-hydroxynaphth-1-yl)carbenium internal salt, monosodium salt (CI44090; Food Green No. 4; Acid Green No. 50), bis[4-(diethylamino)phenyl](2,4-disulfophenyl)carbenium internal salt, sodium salt (2:1) (CI42045; Food Blue No. 3; Acid Blue No. 1), bis[4-(diethylamino)phenyl](5-hydroxy-2,4-disulfophenyl)carbenium internal salt, calcium salt (2:1) (CI42051; Acid Blue No. 3), 1-amino-4-(cyclohexylamino)-9,10-anthraquinone-2-sulfonic acid sodium salt (CI62045; Acid Blue No. 62), 3,3-bis(3,5-dibromo-4-hydroxyphenyl)-4,5,6,7-tetrabromo-2,1(3h)-benzoxathiole 1,1-dioxide, 1-amino-4-(phenylamino)-9,10-anthraquinone-2-sulfonic acid (CI62055; Acid Blue No. 25), 2-(1,3-dihydro-3-oxo-5-sulfo-2H-indol-2-ylidene)-2,3-dihydro-3-oxo-1H-indole-5-sulfonic acid disodium salt (CI73015; Acid Blue No. 74), 9-(2-carboxyphenyl)-3-[(2-methylphenyl)amino]-6-[(2-methyl-4-sulfophenyl)amino]xanthylium internal salt, monosodium salt (CI45190; Acid Violet No. 9), 1-hydroxy-4-[(4-methyl-2-sulfophenyl)amino]-9,10-anthraquinone sodium salt (CI60730; D&C Violet No. 2; Acid Violet No. 43), bis[3-nitro-4-[(4-phenylamino)-3-sulfophenylamino]phenyl]sulfone (CI10410; Acid Brown No. 13), 5-amino-4-hydroxy-6-[(4-nitrophenyl)azo]-3-(phenylazo)-2,7-naphthalenedisulfonic acid disodium salt (CI20470; Acid Black No. 1), 3-hydroxy-4-[(2-hydroxynaphth-1-yl)azo]-7-nitro-1-naphthalenesulfonic acid-chromium complex (3:2) (CI15711; Acid Black No. 52), 3-[(2,4-dimethyl-5-sulfophenyl)azo]-4-hydroxy-1-naphthalenesulfonic acid disodium salt (CI14700; Food Red No. 1; Ponceau SX; FD&C Red No. 4), 4-(acetylamino)-5-hydroxy-6-[(7-sulfo-4-[(4-sulfophenyl)azo]naphth-1-yl)azo]-1,7-naphthalenedisulfonic acid tetrasodium salt (CI28440; Food Black No. 1) and 3-hydroxy-4-(3-methyl-5-oxo-1-phenyl-4,5-dihydro-1H-pyrazol-4-ylazo)naphthalene-1-sulfonicacid sodium salt, chromium complex (Acid Red No. 195).

The colourant **(b)** according to the invention, which is characterized by a content of an oxidizing agent, preferably hydrogen peroxide, besides the dyes of the general formula (I) may additionally also comprise further oxidation-stable direct dyes, such as, for example, 3-(2',6'-diaminopyridyl-3'-azo)pyridine (= 2,6-diamino-3-((pyridin-3-yl)azo)pyridine), N,N-di(2-hydroxyethyl)-3-methyl-4-((4-nitrophenyl)azo)aniline (Disperse Red 17, CI11210), 3-diethylamino-7-(4-dimethylaminophenylazo)-5-phenylphenazinium chloride (CI11050), 4-(2-thiazolylazo)resorcinol, 4-((4-phenylamino)azo)benzosulfonic acid sodium salt (Orange IV), 1-((3-aminopropyl)amino)-9,10-anthracenedione (HC Red No. 8), 3',3",4,5,5',5",6,7-octabromophenol sulfonephthalein (tetrabromophenol Blue), 1-((4-amino-3,5-dimethylphenyl)(2,6-dichlorophenyl)methylene)-3,5-dimethyl-4-imino-2,5-cyclohexadiene-phosphoric acid (1:1) (Basic Blue 77), 3',3",5',5"-tetrabromo-m-cresol sulfonephthalein, 2,4-dinitro-1-naphthol-7-sulfonic acid disodium salt (Acid Yellow 1, CI 10316),4-[2'-hydroxy-1'-naphthyl)azo]benzosulfonic acid sodium salt (Acid Orange 7, CI15510), 3',6'-dihydroxy-2',4',5',7'-tetraiodospiro[isobenzofuran-1(3H), 9'-(9H)xanthen]-3-one disodium salt (Acid Red 51, C145430), 6-hydroxy-5-((2-methoxy-5-methyl-4-sulfophenyl)azo)-2-naphthalenesulfonic acid disodium salt (FD&C Red 40, CI16035), 2,4-dinitro-1-naphthol sodium salt (Acid Yellow 24; C110315), 2',4',5',7'-tetrabromo-4,5,6,7-tetrachloro-3',6'-dihydroxyspiro(isobenzofuran-1(3H), 9'-[9H]xanthen]-3-one disodium salt (Acid Red 92; C145410), 4-(2-hydroxy-1-naphthylazo)-3-methylbenzenesulfonic acid sodium salt (Acid Orange 8, CI15575), 2-amino-1,4-naphthalenedione, dithizone (1,5-diphenylthiocarbazone), N-((2-hydroxyethyl)-2-nitro-4-trifluoromethyl)aniline (HC Yellow 13), N-(2-hydroxyethyl)-4-nitroaniline and 4-chloro-N-(2,3-dihydroxypropyl)-2-nitroaniline, 1-methyl-4-((methylphenylhydrazono)methyl)pyridinium methylsulfate (Basic Yellow No. 87), 3-((4,5-dihydro-3-methyl-5-oxo-1-phenyl-1H-pyrazol-4-yl)azo)-N,N,N-trimethylbenzenaminium chloride, 3-[(3-methyl-5-hydroxy-1-phenyl-1H-pyrazol-4-yl)azo]-trimethylammoniobenzene chloride (Basic Yellow No. 57), 2-((4-aminophenyl)azo)-1,3-dimethyl-1H-imidazol-3-ium chloride (Basic Orange No. 31), 1,4-dimethyl-5-[(4-(dimethylamino)phenyl)azo]-1,2,4-triazolium chloride (Basic Red No. 22, CI11055), 2-((4-(dimethylamino)-phenyl)azo)-1,3-dimethyl-1H-imidazolium chloride (Basic Red No. 51), 1,4-dimethyl-5-[[4-[methyl(phenylmethyl)amino]phenyl]-azo]-1,2,4-triazolium bromide (Basic Red No. 46), N,N,N-trimethyl-3-{[4-(methylamino)-9,10-dioxo-9,10-dihydro-1-anthracenyl]amino}-1-propanaminium methylsulfate, N,N-dimethyl-3-{[4-(methylamino)-9,10-dioxo-9,10-dihydro-1-anthracenyl]amino}-N-propyl-1-propanaminium chloride and N,N-dimethyl-3-{[4-(methylamino)-9,10-dioxo-9,10-dihydro-1-anthracenyl]amino}-N-propyl-1-propanaminium bromide.

The total content of additional dyes in the colourant according to the invention is about 0.01 to 15% by weight, in particular 0.1 to 12% by weight.

The oxidation colourant (c) according to the invention which is mixed prior to application with an oxidizing agent (in particular hydrogen peroxide or its addition compounds) comprises, besides the dyes of the general formula (I), oxidation dye precursors and if necessary one or more of the abovementioned direct dyes, provided these direct dyes are stable to the oxidizing agent used.

Suitable oxidation dye precursors which may be specified are, for example, the following developer substances and coupler substances and self-coupling compounds:
(i) Developer substances: 1,4-diaminobenzene (p-phenylenediamine), 1,4-diamino-2-methylbenzene (p-tolylenediamine), 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)benzene, 2-(2-(acetylamino)ethoxy)-1,4-diaminobenzene, 4-phenylaminoaniline, 4-dimethylaminoaniline, 4-diethylaminoaniline, 4-dipropylaminoaniline, 4-[ethyl(2-hydroxyethyl)-amino]aniline, 4-[di(2-hydroxyethyl)amino]aniline, 4-[di(2-hydroxyethyl)-amino]-2-methylaniline, 4-[(2-methoxyethyl)amino]aniline, 4-[(3-hydroxypropyl)amino]aniline, 4-[(2,3-dihydroxypropyl)amino]aniline, 1,4-diamino-2-(2-hydroxyethyl)benzene, 1,4-diamino-2-(1-methylethyl)-benzene, 1,3-bis[(4-aminophenyl)(2-hydroxyethyl)amino]-2-propanol, 1,4-bis[(4-aminophenyl)amino]butane, 1,8-bis(2,5-diaminophenoxy)-3,6-dioxaoctane, 4-aminophenol, 4-amino-3-methylphenol, 4-amino-3-(hydroxymethyl)phenol, 4-amino-3-fluorophenol, 4-methylaminophenol, 4-amino-2-(aminomethyl)phenol, 4-amino-2-(hydroxymethyl)phenol, 4-amino-2-fluorophenol, 4-amino-2-[(2-hydroxyethyl)amino]methylphenol, 4-amino-2-methylphenol, 4-amino-2-(methoxymethyl)phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 1-[(4-chlorophenyl)methyl]-4,5-diamino-1H-pyrazole, 4,5-diamino-1-methyl-1H-pyrazole, 2-aminophenol, 2-amino-6-methylphenol, 2-amino-5-methylphenol, alone or in a mixture with one another.
(ii) Coupler substances: N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino] aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)-amino]aniline, 1,3-di(2,4-diaminophenoxy)propane, di(2,4-diamino-phenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)-amino]-2-methylphenol, 3-[(2-hydroxyethyl)amino]phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)-amino]-2-methylphenol, 3-[(2-hydroxyethyl)amino]-2-methylphenol, 2-amino-3-hydroxypyridine, 5-amino-4-chloro-2-methylphenol, 1-naphthol, 2-methyl-1-naphthol, 1,5-dihydroxynaphthalene, 1,7-dihydroxy-naphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole, 2,3-indolenedione, alone or in a mixture with one another.
(iii) Self-coupling compounds: 2-amino-5-methylphenol, 2-amino-5-ethylphenol, 2-amino-6-methylphenol, 2-amino-5-ethoxyphenol or 2-propylamino-5-aminopyridine.

The total amount of the oxidation dye precursors present in the colourant (c) according to the invention is from about 0.01 to 12% by weight, in particular from about 0.2 to 6% by weight.

To increase the colour intensity, the carriers customary used in cosmetic systems can be added if required. Suitable compounds are described, for example, in DE-A 196 18 595, which is herewith expressly included by reference. Particularly suitable carriers are, for example, benzyl alcohol, vanillin and isovanillin.
For colouring, the dyes described above are applied in a suitable colour carrier mass.

The colourant **(a), (b)** or **(c)** according to the invention can also comprise all additives customary and known for such preparations, for example perfume oils, complexing agents, waxes, preservatives, thickeners, antioxidants, alginates, guar gum, haircare substances, such as, for example, cationic polymers or lanolin derivatives, or anionic, nonionic, amphoteric or cationic surface-active substances. Preference is given to using amphoteric or nonionic surface-active substances, for example betaine surfactants, propionates and glycinates, such as, for example, cocoamphoglycinates or cocoamphodiglycinates, ethoxylated surfactants with 1 to 1000 ethylene oxide units, preferably with 1 to 300 ethylene oxide units, such as, for example, glyceride alkoxylates, for example castor oil ethoxylated with 25 ethylene oxide units, polyglycolamides, ethoxylated alcohols and ethoxylated fatty alcohols (fatty alcohol alkoxylates) and ethoxylated fatty acid sugar esters, in particular ethoxylated sorbitan fatty acid esters. The abovementioned constituents are used in the amounts customary for such purposes, for example the surface-active substances in a concentration of from 0.1 to 30% by weight, and the care substances in an amount of from 0.1 to 5% by weight.

The colourant **(a), (b)** or **(c)** according to the invention, particularly if it is a hair colourant, can be present in the form of a powder or granules which is/are dissolved prior to application in an aqueous or aqueous-alcoholic preparation, or else in the form of an aqueous or aqueous-alcoholic solution, a cream, a gel, an emulsion or an aerosol foam,
where the colourant can be formulated either in the form of a single-component preparation or else in the form of a multicomponent preparation, for example in the form of a two-component preparation in which the particular dye derivative of the general formula (I) is packaged separately from the other constituents and the ready-to-use colourant is only prepared directly prior to application by mixing the two components.

The colourant **(a), (b)** or **(c)** according to the invention generally has a pH of from about 2 to 11, preferably from about 5 to 10. Both organic and inorganic acids or bases are suitable for adjusting the pH according to the invention.
Examples of suitable acids are, in particular, the following acids: α-hydroxycarboxylic acids, such as, for example, glycolic acid, lactic acid, tartaric acid, citric acid or malic acid, ascorbic acid, gluconic acid lactone, acetic acid, hydrochloric acid or phosphoric acid, and mixtures of these acids.
Examples of suitable bases are, in particular, sodium carbonate, sodium hydrogencarbonate, organic amines, for example monoethanolamine, triethanolamine, 2-amino-2-methyl-1-propanol or tris(hydroxymethyl)aminomethane, ammonia, potassium hydroxide or sodium hydroxide, and mixtures thereof.

Depending on the intended use, the colourant according to the invention can be used with one or more oxidizing agents (lightening; oxidation colourants) or without an oxidizing agent (nonoxidative colourants).

The compositions according to the present invention may comprise at least one source of an oxidizing agent for developing the hair colour. Preferred oxidizing agents for use herein are water-soluble peroxygen oxidizing agents. "Water-soluble" as defined herein means that in standard condition at least 0.1g, preferably 1g, more preferably 10g of said oxidizing agent can be dissolved in 1 liter of deionized water. The oxidizing agents are valuable for the initial solubilisation and decolourisation of the melanin (bleaching) and accelerate the oxidation of the oxidative dye precursors (oxidative dyeing) in the hair shaft.

Any oxidizing agent known in the art may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Alkyl and aryl peroxides, and or peroxidases may also be used. Mixtures of two or more such oxidizing agents can also be used if desired. The oxidizing agents may be provided in aqueous solution or as a powder which is dissolved prior to use. Preferred for use in the compositions according to the present invention are hydrogen peroxide, percarbonate, persulphates and combinations thereof.

According to the present invention the compositions comprise from about 0.1% to about 15% by weight, preferably from about 1% to about 10% by weight, and most preferably from about 2% to about 7% by weight of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 9.5, preferably 7.5 to 9.5 more preferably about pH 9. Moreover, this system is also particularly effective in combination with a source of ammonia or ammonium ions. It has been found that this oxidizing agent can deliver improvements to the desired hair colour results particularly with regard to the delivery of high lift, whilst considerably reducing the odour, skin and scalp irritation and damage to the hair fibres.

Accordingly, any source of these ions may be utilized. Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

According to the present invention the compositions comprise from about 0.1% to about 15% by weight, preferably from about 1 % to about 10% by weight, and most preferably from about 1 % to about 8% by weight of a hydrogencarbonate ion and from about 0.1% to about 10% by weight, preferably from about 1 % to about 7% by weight, and most preferably from about 2% to about 5% by weight of a source of hydrogen peroxide.

Especially preferred oxidants for developing the hair colour are mainly hydrogen peroxide or a compound of addition of hydrogen peroxide to urea, melamine, sodium borate or sodium carbonate, in the form of a 3 to 12%, preferably 6%, aqueous solution, as well as air oxygen. When a 6% hydrogen peroxide solution is used as the oxidant, the weight ratio of hair colourant to oxidant is 5:1 to 2:1, and preferably 1:1. Larger amounts of oxidant are used primarily when the hair colourant contains a higher dye concentration or when stronger hair bleaching is desired at the same time.
According to the present invention the compositions may further comprise a source of radical scavenger. As used herein the term radical scavenger refers to a species that can react with a reactive radical, preferably carbonate radicals, to convert the reactive radical by a series of fast reactions to a less reactive species. Suitable radical scavengers for use herein include compounds according to the general formula (RSI):

R^{1'}-Y-C(H)(R³)-R^{4'}-(C(H)(R^{5'})-Y-R^{6'})ₙ (RSI)

wherein Y is NR^{2'}, O, or S, preferably NR^{2'}, n is 0 to 2, and wherein R^{4'} is monovalent or divalent and is selected from: (a) substituted or unsubstituted, straight or branched, alkyl, mono- or poly-unsaturated alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; the systems of (a), (b) and (c) comprising from 1 to 12 carbon atoms and 0 to 5 heteroatoms selected from O, S, N, P, and Si; and wherein R⁴ can be connected to R^{3'} or R^{5'} to create a 5, 6 or 7 membered ring; and wherein R^{1'}, R^{2'}, R^{3'}, R^{5'}, and R^{6'} are monovalent and are selected independently from: (a), (b) and (c) described herein above, or H.
Preferably, R^{4'} is selected from: (a) substituted or unsubstituted, straight or branched, alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; more preferably R^{4'} is selected from (a) substituted or unsubstituted, straight or branched, alkyl, heteroalkyl, aliphatic, or heteroaliphatic systems, (b) substituted or unsubstituted, aryl, or heterocyclic systems, or (c) substituted or unsubstituted, mono-, poly-, or per-fluoro alkyl systems; more preferably substituted or unsubstituted, straight or branched, alkyl, or heteroalkyl systems.
Preferably, the R^{4'} systems of (a), (b), and (c), described herein above, comprise from 1 to 8 carbon atoms, preferably from 1 to 6, more preferably from 1 to 4 carbon atoms and from 0 to 3 heteroatoms; preferably from 0 to 2 heteroatoms; most preferably from 0 to 1 heteroatoms. Where the systems contain heteroatoms, preferably they contain 1 heteroatom. Preferred heteroatoms include O, S, and N; more preferred are O, and N; O being paricularly preferred.
Preferably, R^{1'}, R^{2'}, R^{3'}, R^{5'}, and R^{6'} are selected independently from any of the systems defined for R^{4'} above, and H.
In alternative embodiments, any of R^{1'}, R^{2'}, R^{3'}, R^{4'}, R^{5'}, and R^{6'} groups are substituted. Preferably, the substituent(s) is selected from: (a) the group of C-linked monovalent substituents consisting of: (i) substituted or unsubstituted, straight or branched, alkyl, mono- or poly-unsaturated alkyl, heteroalkyl, aliphatic, heteroaliphatic, or heteroolefinic systems, (ii) substituted or unsubstituted, mono- or poly-cyclic aliphatic, aryl, or heterocyclic systems, or (iii) substituted or unsubstituted, monofluoroalkyl, polyfluoroalkyl or perfluoroalkyl systems; said systems of (i), (ii) and (iii) comprising from 1 to 10 carbon atoms and 0 to 5 heteroatoms selected from O, S, N, P, and Si; (b) the group of S-linked monovalent substituents consisting of SA¹, SCN, SO₂A¹, SO₃A¹, SSA¹, SOA¹, SO₂NA¹A², SNA¹A², and SONA¹A²; (c) the group of O-linked monovalent substituents consisting of OA¹, OCN and ONA¹A²; (d) the group of N-linked monovalent substituents consisting of NA¹A², (NA¹A²A³)⁺,NC, NA¹OA², NA¹SA², NCO, NCS, NO₂, N=NA¹, N=NOA¹, NA¹CN, NA¹NA²A³; (e) the group of monovalent substituents consisting of COOA¹, CON₃, CONA¹₂, CONA¹COA², C(=NA¹)NA¹A², CHO, CHS, CN, NC, and X; and (f) the group consisting fluoroalkyl monovalent substituents consisting of monofluoroalkyl, polyfluoroalkyl perfluoroalkyl systems comprising from 1 to 12 carbon atoms and 0 to 4 heteroatoms.
For the groups (b) to (e), described above, A¹, A², and A³ are monovalent and are independently selected from: (1) H, (2) substituted or unsubstituted, straight or branched, alkyl, monounsaturated or poly-unsaturated alkyl, heteroalkyl, aliphatic, heteroaliphatic or heteroolefinic systems,
(3) substituted or unsubstituted, monocyclic or polycyclic aliphatic, aryl or heterocyclic systems, or (4) substituted or unsubstituted, monofluoroalkyl, polyfluoroalkyl or perfluoroalkyl systems; said systems of (2), (3) and (4) comprising from 1 to 10 carbon atoms and 0 to 5 heteroatoms selected from O, S, N, P, and Si; and wherein X is a halogen selected from the group consisting of F, Cl, Br, and I.
Preferred substituents for use herein include those having a Hammett Sigma Para (σₚ) Value from -0.65 to +0.75, preferably from -0.4 to +0.5. Hammett Sigma Values are described in Advanced Organic Chemistry - Reactions, Mechanisms and Structure (Jerry March, 5th ed. (2001) at pages 368-375).

Alternative suitable radical scavengers for use herein are compounds according to the general formula (RSII) : 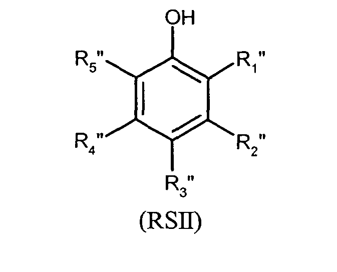 wherein R_{1"}, R_{2"}, R_{3"}, R_{4"}, and R_{5"} are each independently selected from H, COO⁻M⁺, Cl, Br, SO₃⁻M⁺, NO₂, OCH₃, OH or a Cl to C10 primary or secondary alkyl and M is either H or alkali metal. Preferably, the above-described radical scavengers have a pKa of more than 8.5 to ensure protonation of the hydroxy goup.

Other suitable radical scavengers for use herein include those selected from group (RSIII) benzylamine, imidazole, di-tert-butylhydroxytoluene, hydroquinone, guanine, pyrazine, piperidine, morpholine, methylmorpholine, 2-methyoxyethylamine, and mixtures thereof. Preferred radical scavengers according to the present invention are selected from the classes of alkanolamines, amino sugars, amino acids, esters of amino acids and mixtures thereof. Particularly preferred compounds are: monoethanolamine, 3-amino-1-propanol, 4-amino-1-butanol, 5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof. Especially preferred compounds are glycine, sarcosine, lysine, serine, 2 methoxyethylamine, glucosamine, glutamic acid, morpholine, piperdine, ethylamine, 3 amino-1-propanol and mixtures thereof.

The radical scavengers according to the present invention preferably have a molecular weight of less than about 500, preferably less than about 300, more preferably less than about 250 in order to facilitate penetration of the radical scavenger into the hair fibre. The compositions of the present invention preferably comprise from about 0.1 % to about 10% by weight, preferably from about 1 % to about 7% by weight of radical scavenger. The radical scavenger is also preferably selected such that it is not an identical species as the alkalizing agent. According to one embodiment of the present invention the radical scavenger may be formed insitu in the hair dyeing compositions prior to application to the hair fibres.

To use the afore-described colourants for oxidative dyeing of hair, said colourants are mixed with an oxidant immediately before use, and the mixture is applied to hair in an amount sufficient for hair.

Suitable oxidants for developing the hair colour are mainly hydrogen peroxide or a compound of addition of hydrogen peroxide to urea, melamine, sodium borate or sodium carbonate, in the form of a 3 to 12% by weight, preferably 6% by weight, aqueous solution, as well as air oxygen (with or without the addition of enzymes). When a 6% hydrogen peroxide solution is used as the oxidant, the weight ratio of hair colourant to oxidant is 5:1 to 2:1, and preferably 1:1. Larger amounts of oxidant are used primarily when the hair colourant contains a higher dye concentration or when stronger hair bleaching is desired at the same time.

The colourant according to the invention is generally used by applying an amount of the hair colourant sufficient for the hair colouring, about 30 to 200 grams depending on hair length, to the hair, allowing the hair colourant to act at about 15 to 50 degrees Celsius for about 1 to 60 minutes, preferably 5 to 30 minutes, then rinsing the hair thoroughly with water, optionally washing with a shampoo and/or after-treating with a hair-conditioning composition and finally drying.

In addition, if no oxidizing agents are added to the colouring mass, the above-described colourant can comprise natural or synthetic polymers or modified polymers of natural origin customary for cosmetic compositions, as a result of which setting of the hair is achieved at the same time as the colouring. Such compositions are generally referred to as colour setting compositions.

Suitable synthetic polymers known for this purpose in cosmetics, are, for example, polyvinylpyrrolidone, polyvinyl acetate, polyvinyl alcohol or polyacrylic compounds, such as polyacrylic acid or polymethacrylic acid, basic polymers of esters of polyacrylic acid, basic polymers of polymethylacrylic acid and amino alcohols, for example salts or quatemization products thereof, polyacrylonitrile, polyvinyl acetates, and copolymers of such compounds, such as, for example, polyvinylpyrrolidone-vinyl acetate; while natural polymers or modified natural polymers which can be used are, for example, chitosan (deacetylated chitin) or chitosan derivatives.

The abovementioned polymers may be present in the colourant (a) according to the invention in the amounts customary for such agents, in particular in an amount of from about 1 to 5% by weight. The pH of the colour setting composition or colour setting composition according to the invention is preferably about 4 to 10.

The hair colourant with additional setting properties is used in a known and customary manner by wetting the hair with the setting composition, arranging (styling) the hair into the hairstyle and then drying.

The colourant according to the invention permits a colouration of keratin fibres, in particular of human hair, with a very strong colour intensity and brilliance, and a very good durability (washing fastness), specially when applied together with hydrogen peroxide to natural hair or damaged hair (e.g. bleached or permed hair).
The zwitterionic azomethine dyes according to the invention of the general formula (I), (II) or (III) can be prepared via a 2-step process, by condensation of aromatic or heteroaromatic hydrazine or the acid addition salt thereof (commercialy available or accessible via standard literature procedure) with heterocyclic aldehydes or ketones. The resulting azomethine dye is converted to the zwitterionic azomethine dye of the formula (I), (II) or (III) using sultones, such as, for example, butane sultone or propane sultone (Diagram 1). 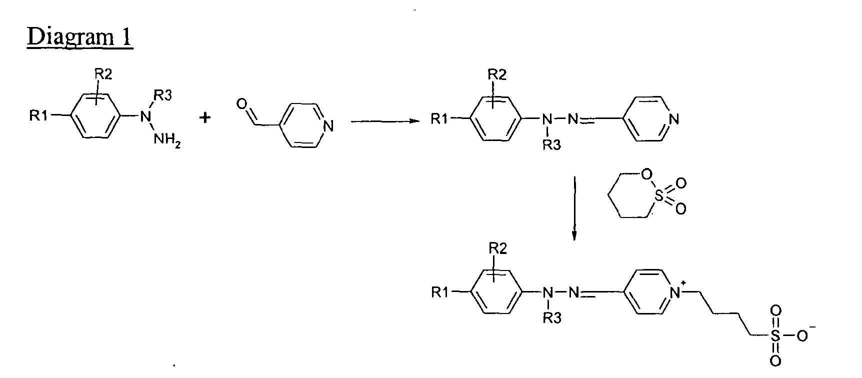 Another preparation process is a one-step process, by the condensation of an aromatic or heteroaromatic hydrazine with a previously prepared zwitterionic aldehyde or ketone (Diagram 2).

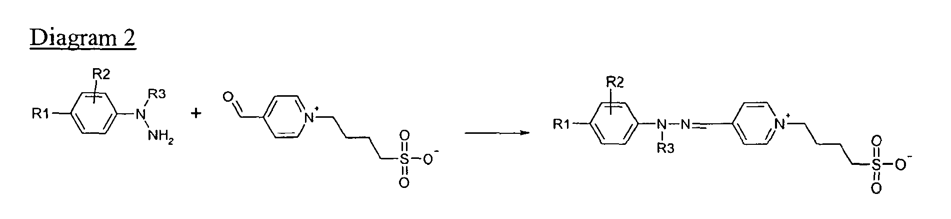

The examples below are intended to illustrate the subject-matter of the invention in more detail without limiting it thereto.

### Examples

### Example 1: Synthesis of azomethine butanesulfonates of formula (I)

### Example 1a: Synthesis of 4-(4-{[methyl(phenyl)hydrazono]methyl}-1-pyrridiniumyl)-1-butanesulfonate

4.5 g (21.3 mmol) isonicotinaldehyde methyl(phenyl)hydrazone were heated under stirring with 50 ml butane sultone at 110 °C for 2 hours. After cooling, the resulting precipitate was filtered off and washed with acetone. The cake was triturated in acetone, filtered off, and dried.
6.9 g (93% of theory) 4-(4-{[methyl(phenyl)hydrazono]methyl}-1-pyridiniumyl)-1-butanesulfonate were obtained as a bright yellow powder.
¹H-NMR (300 MHz, DMSO): δ =8.83 (d, J=6.3, 2H, H(2) and H(6) pyridinium); 8.16 (d, J=6.3, 2H, H(3) and H(5)-pyridinium); 7.80 (s, 1H, azomethine); 7.60 (d, J=7.8, 2H, H(2) and H(6)-phenyl); 7.41 (m, 2H, H(3) and H(5)-phenyl); 7.12 (m, 1H, H(4)-phenyl); 4.50 (t, J=7.5, 2H, N⁺CH2); 3.59 (s, 3H, N-CH3); 2.48 (t, J=7.5, 2H, CH2-SO3⁻); 1.99 (quintett, J=7.5, 2H, CH2); 1.58 (quintett, J=7.5, 2H, CH2).
API-ES MS: 370 [M⁺+ Na] (100)

### Example 1b: Synthesis of 4-(3-{[methyl(phenyl)hydrazono]methyl}-1-pyridiniumyl)-1-butanesulfonate

Analogously to the process described in example 1a, 4-(3-{[methyl(phenyl)hydrazono]-methyl}-1-pyridiniumyl)-1-butanesulfonate was prepared from 3-{[methyl(phenyl)-hydrazono]methyl}pyridine in 95% yield.
¹H-NMR (300 MHz, DMSO): δ = 9.28 (s, 1H, H(2)-pyridinium); 8.90 (d, J=6.3, 1H, H(6)-pyridinium); 8.74 (d, J=7.8, 1H, H(4)-pyridinium); 8.10 (dd, J=6.3, J=7.8, 1H, H(5)-pyridinium); 7.75 (s, 1H, azomethine); 7.56 (d, J=8.7, 2H, H(2) and H(6)-phenyl); 7.37 (m, 2H, H(3) and H(5)-phenyl); 7.02 (m, 1H, H(4)-phenyl); 4.67 (t, J=7.5, 2H, N⁺CH2); 3.51 (s, 3H, N-CH3); 2.48 (t, J=7.5, 2H, CH2-SO3⁻); 2.09 (quintett, J=7.5, 2H, CH2); 1.61 (quintett, J=7.5, 2H, CH2).
API-ES MS: 370 [M⁺+Na] (100)

### Example 1c: Synthesis of 4-(2-{[methyl(phenyl)hydrazono]methyl}-1-pyridiniumyl)-1-butanesulfonate

Analogously to the process described in example 1a, 4-(2-{[methyl(phenyl)hydrazono]-methyl}-1-pyridiniumyl)-1-butanesulfonate was prepared from 2-{[methyl(phenyl)-hydrazono]methyl}pyridine in 65% yield.
¹H-NMR (300 MHz, DMSO): δ = 8.81 (d, J=6.3, 1H, H(6)-pyridinium); 8.57 (d, J=8.7, 1H, H(3)-pyridinium); 8.35 (m, 1H, H(4)- pyridinium); 7.85 (s, 1H, azomethine); 7.80 (dd, J=6,3, J=7.5, 1H, H(5)-pyridinium); 7.61 (d, J=8.4, 2H, H(2) and H(6)-phenyl); 7.43 (m, 2H, H(3) and H(5)-phenyl); 7.15 (m, 1H, H(4)-phenyl); 4.78 (t, J=7.5, 2H, N⁺CH2); 3.72 (s, 3H, N-CH3); 2.54 (t, J=7.5, 2H, CH2-SO3⁻); 2.05 (quintett, J=7.5, 2H, CH2); 1.75 (quintett, J=7.5, 2H, CH2).
API-ES MS: 370 [M⁺+ Na] (100)

### Example 1d: Synthesis of 4-[4-{[4-(methoxy)phenyl]hydrazono}methyl)-1-pyridiniumyl]-1-butanesulfonate

Analogously to the process described in example 1a, 4-[4-({[4-(methoxy)phenyl]-hydrazono}methyl)-1-pyridiniumyl]-1-butanesulfonate was prepared from isonicotinaldehyde (4-methoxyphenyl)hydrazone in 98 % yield.
¹H-NMR (300 MHz, DMSO): δ = 8.76 (d, J=6.3, 2H, H(2) and H(6) pyridinium); 8.11 (d, J=6.3, 2H, H(3) and H(5)-pyridinium); 7.82 (s, 1H, azomethine); 7.60 (d, J=7.8, 2H, H(2) and H(6)-phenyl); 6.95 (d, J=8.7, 2H, H(3) and H(5)-phenyl); 4.46 (t, J=7.5, 2H, N⁺CH2); 3.74 (s, 3H, OCH3); 2.46 (t, J=7.5, 2H, CH2-SO3⁻); 1.99 (quintett, J=7.5, 2H, CH2); 1.58 (quintett, J=7.5, 2H, CH2).
API-ES MS: 386 [M⁺+ Na] (100)

### Example 1e: Synthesis of 4-[4-({methyl[4-(methoxy)phenyl]hydrazono}methyl)-1-pyridiniumyl]-1-butanesulfonate

To a solution of 0.5 g (1.38 mmol) 4-[4-{[4-(methoxy)phenyl]hydrazono}methyl)-1-pyridiniumyl]-1-butanesulfonate (example 1d) in 10 ml N-methylpyrolidine were added 0.77 g (13.8 mmol) potassium hydroxide and 1.96 g (13.8 mmol) methyliodide. The reaction mixture was stirred overnight at room temperature. 200 ml acetone were added to the reaction mixture and the resulting precipitate was filtered off, washed with ethylacetate, and dried under vacuum.
0.36 g (69%) 4-[4-({methyl[4-(methoxy)phenyl]hydrazono}methyl)-1-pyridiniumyl]-1-butanesulfonate were obtained as an orange powder.
¹H-NMR (300 MHz, DMSO): δ = 8.78 (d, J=6.3, 2H, H(2) and H(6)-pyridinium); 8.09 (d, J=6.3, 2H, H(3) and H(5)-pyridinium); 7.69 (s, 1H, azomethine); 7.51 (d, J=9.0, 2H, H(2) and H(6)-phenyl); 6.98 (d, J=9.0, 2H, H(3) and H(5)-phenyl); 4.47 (t, J=7.2, 2H, N⁺CH2); 3.77 (s, 3H, OCH3); 3.57 (s, 3H, NCH3); 2.51 (t, J=7.5, 2H, CH2-SO3⁻); 1.98 (quintett, J=7.5, 2H, CH2); 1.58 (quintett, J=7.5, 2H, CH2).
API-ES MS: 400 [M⁺+ Na] (100)

### Example 1f: Synthesis of 4-(2-{[methyl(phenyl)hydrazono]methyl}-1,3-thiazol-3-ium-3-yl)-1-butanesulfonate

Analogously to the process described in example 1a, 4-(2-{[methyl(phenyl)-hydrazono]methyl}-1,3-thiazol-3-ium-3-yl)-1-butanesulfonate was prepared from 2-{[methyl(phenyl)hydrazono]methyl}-1,3-thiazole in 52 % yield.
¹H-NMR (300 MHz, DMSO): δ = 8.28 (d, J=3.9, 1H, H(4)-thiazolium); 8.11 (s, 1H, azomethine); 8.00 (d, J=3.9, 1H, H(5)-thiazolium); 7.54-7.43 (m, 4H, H-phenyl); 7.20 (m, 1H, H(4)-phenyl); 4.60 (t, J=7.5, 2H, N⁺CH2); 3.70 (s, 3H, N-CH3); 2.53 (t, J=7.5, 2H, CH2-SO3⁻); 1.97 (quintett, J=7.5, 2H, CH2); 1.68 (quintett, J=7.5, 2H, CH2). API-ES MS: 376 [M⁺+ Na] (100)

### Example 2: Synthesis of azomethine butanesulfonates of formula (II)

### Example 2a: Synthesis of 4-(4-{[2-(3-methyl-1,3-benzothiazol-2(3H)-ylidene)hydrazono]methyl}-1-pyridiniumyl)-1-butanesulfonate

0,3 g (1,12 mmol) isonicotinaldehyde-3-methyl-1,3-benzothiazol-2(3*H*-ylidene)-hydrazone were dissolved in 10 ml of dry 3-methoxypropanenitrile and heated under stirring to 100°C. Then 150 mg (1,12 mmol) butane sultone was added dropwise while the mixture turned orange. Then the oil bath temperature was rised to 150 °C and stirring was continued. After 4 h the reaction mixture was allowed to cool to 0 °C in an ice bath and the resulting precipitate was filtered off, washed with cold aceton and dried under vacuum.
0,32 g (71 %) 4-(4-{[2-(3-methyl-1,3-benzothiazol-2(3*H*)- ylidene)hydrazono]methyl}-1-pyridiniumyl)-1-butanesulfonate were obtained as an orange powder.
¹H-NMR (DMSO/300 MHz): δ = 9,00 (d, 2H, *J=* 6,3, pyridinium); 8,51 (s, 1H, azomethine); 8,25 (d, 2H, *J=* 6,3, pyridinium); 7,76 (d, 1H, benzothiazole); 7,44-7,49 (m, 2H, benzothiazole); 7,24 (t, 1H, benzothiazole); 4,57 (t, 2H, CH₂); 3,71 (s, 3H, CH₃); 2,46 (t, 2H, CH₂); 2,02 (quintett, 2H, CH₂); 1,61 (quintett, 2H, CH₂).

### Example 2b: Synthesis of 4-(3-{[2-(3-methyl-1,3-benzothiazol-2(3H)-ylidene)hydrazono]methyl}-1-pyridiniumyl)-1-butanesulfonate

Analogously to the process described in example 2a, 4-(3- {[2-(3-methyl-1,3-benzothiazol-2(3*H*)-ylidene)hydrazono]methyl}-1-pyridiniumyl)-1-butanesulfonate was prepared from nicotinaldehyde-(3-methyl-1,3-benzothiazol-2(3*H*)-ylidene)hydrazone in 87 % yield.
¹H-NMR (DMSO/300 MHz): δ = 9,32 (s, 1H, pyridinium); 9,05 (d, 1H, pyridinium); 8,79 (d, 1H, pyridinium); 8,49 (s, 1H, azomethine); 8,18 (t, 1H, pyridinium); 7,70 (d, 1H, benzothiazole); 7,42-7.40 (m, 2H, benzothiazole); 7,21-7,16 (m, 1H, benzothiazole); 4,68 (t, 2H, CH₂); 3,65 (s, 3H, CH₃); 2,50 (t, 2H, CH₂); 2,07 (quintett, 2H, CH₂); 1,63 (quintett, 2H, CH₂).

### Example 2c: Synthesis of 4-(4-{[2-(3,4-dimethyl-1,3-thiazol-2(3H)-ylidene)hydrazono]methyl}-1-pyridiniumyl)-1-butanesulfonate

Analogously to the process described in example 2a, 4-(4-{[2-(3,4-dimethyl-1,3-thiazol-2(3*H*)-ylidene)hydrazono]methyl}-1-pyridiniumyl)-1-butanesulfonate was prepared (3,4-dimethyl-1,3-thiazol-2(3*H*)-ylidene)hydrazone in 85 % yield.
¹H-NMR (DMSO/300 MHz): δ = 8,87 (d, 2H, *J=* 6,3, pyridinium); 8,30 (s, 1H, azomethine); 8,13 (d, 2H, *J*= 6,3, pyridinium); 6,46 (s, 1H, thiazole); 4,51 (t, 2H, CH₂); 3,43 (s, 3H, CH₃); 2,56-2,45 (m, 5H, CH₂, CH₃, overlap); 1,96 (m, 2H, CH₂); 1,58 (m, 2H, CH₂).

**Example 3 to 11:** Hair colourant

| | |
|---|---|
| 2.5 mmol | Dye of the formula (I), (II) or (III) |
| 5.0 g | Ethanol |
| 4.0 g | Decyl glucoside |
| 0.2 g | Ethylenediaminotetraacetic acid disodium salt |
| ad 100.0 g | Water, demineralized |

If necessary, the colouring solution is adjusted to the pH values given in table 1 by adding 25% ammonia.

The hair colouring is carried out by applying an amount of the colourant sufficient for the hair colouring to the hair and distributing it evenly using a brush. After a contact time of 30 minutes at 40 °C, the hair is rinsed with lukewarm water, washed with a shampoo, rinsed with lukewarm water and then dried.
The colouring results are summarized in table 1 below.

**Table 1:**

| **Ex.** | **Compound of the formula (I), (II) or (III) (as in examples 1 to 2)** | **pH of the colourant** | **Colour shade after colouring** | **Colour measurement values after colouring** |
|---|---|---|---|---|
| **-** | Colour shade of the hair before the colouring treatment | --- | --- | L= 80.60 |
| | | | | C= 12.10 |
| | | | | h= 92.10 |
| **3** | 4-(4-{[methyl(phenyl)hydrazono]-methyl}-1-pyridiniumyl)-1-butanesulfonate (1a) | 7.3 | bright | L= 78.16 |
| | | | golden | C= 91.84 |
| | | | yellow | h= 92.70 |
| **4** | 4-(3-{[methyl(phenyl)hydrazono]-methyl}-1-pyridiniumyl)-1-butanesulfonate (1b) | 7.7 | Bright | L= 80.21 |
| | | | lemon | C= 67.67 |
| | | | yellow | h= 98.10 |
| **5** | 4-(2-{[methyl(phenyl)hydrazono]-methyl}-1-pyridiniumyl)-1-butanesulfonate (1c) | 7.4 | Bright | L= 80.41 |
| | | | lemon | C= 72.35 |
| | | | yellow | h= 99.20 |
| **6** | 4-[4-({[4-(methoxy)phenyl]-hydrazono}methyl)-1-pyridiniumyl]-1-butanesulfonate (1d) | 6.0 | Bright | L= 64.39 |
| | | | orange | C= 77.09 |
| | | | | h= 67.10 |
| **7** | 4-[4-({methyl[4-(methoxy)phenyl]-hydrazono}methyl)-1-pyridiniumyl]-1-butanesulfonate (1e) | 7.3 | Bright | L= 61.40 |
| | | | orange- | C= 74.69 |
| | | | yellow | h= 72.50 |
| **8** | 4-(2-{[methyl(phenyl)hydrazono]-methyl}-1,3-thiazol-3-ium-3-yl)-1-butanesulfonate (1f) | 6.0 | Bright | L= 81.13 |
| | | | lemon | C= 81.32 |
| | | | yellow | h= 97.20 |
| **9** | 4-(4-{[2-(3-methyl-1,3-benzothiazol-2(3*H*)-ylidene)hydrazono]methyl}-1-pyridiniumyl)-1-butanesulfonate (2a) | 7.3 | orange | L= 73.90 |
| | | | | C= 56.22 |
| | | | | h= 80.00 |
| **10** | 4-(3-{[2-(3-methyl-1,3-benzothiazol-2(3*H*)-ylidene)hydrazono]methyl}-1-pyridiniumyl)-1-butanesulfonate (2b) | 7.2 | lemon | L= 78.49 |
| | | | yellow | C= 51.92 |
| | | | | h= 95.90 |
| **11** | 4-(4-{[2-(3,4-dimethyl-1,3-thiazol-2(3*H*)-ylidene)hydrazono]methyl}-1-pyridiniumyl)-1-butanesulfonate (2d) | 6.8 | red | L= 43.66 |
| | | | | C= 62.88 |
| | | | | h= 37.80 |

### Example 12: Hair colourant with cationic surface-active substances

| | |
|---|---|
| 0.87 g | 4-(4-{[methyl(phenyl)hydrazono]methyl}-1-pyridiniumyl)-1-butanesulfonate (1a) |
| 5.00 g | Ethanol |
| 4.00 g | Cetyltrimethylammonium chloride, 25% in water |
| ad 100.00 g | Water, demineralized |

The pH is adjusted to the pH values given in table 2 using 25% ammonia.

The hair colouring is carried out by applying an amount of the colourant sufficient for the hair colouring to the hair and distributing it evenly using a brush. After a contact time of 30 minutes at 40 °C, the hair is rinsed with lukewarm water, washed with a shampoo, rinsed again with lukewarm water and then dried.

The colouring results are summarized in table 2 below.

**Table 2:**

| **Ex.** | **Compound of the formula (I)** | **pH of the colourant** | **Colour shade after colouring** | **Colour measurement values after colouring** |
|---|---|---|---|---|
| **-** | Colour shade of the hair before the colouring treatment | --- | --- | L= 80.60 |
| | | | | C= 12.10 |
| | | | | h= 92.10 |
| **12** | 4-(4-{[methyl(phenyl)hydrazono]-methyl}-1-pyridiniumyl)-1-butanesulfonate (1a) | 9.3 | Bright | L= 78.95 |
| | | | golden | C= 92.05 |
| | | | yellow | h= 93.60 |

### Example 13: Hair colourant with amphoteric surface-active substances

| | |
|---|---|
| 0.87 g | 4-(4-{[methyl(phenyl)hydrazono]methyl}-1-pyridiniumyl)-1-butanesulfonate (1a) |
| 5.00 g | Ethanol |
| 7.50 g | Coconut fatty acid amidopropylbetaine |
| ad 100.00 g | Water, demineralized |

The pH is adjusted to the pH values given in table 3 using 25% ammonia. The hair colouring is carried out by applying an amount of the colourant sufficient for the hair colouring to the hair and distributing it evenly using a brush. After a contact time of 30 minutes at 40 °C, the hair is rinsed with lukewarm water, washed with a shampoo, rinsed again with lukewarm water and then dried.

The colouring results are summarized in table 3 below.

**Table 3:**

| **Ex.** | **Compound of the formula (I)** | **pH of the colourant** | **Colour shade after colouring** | **Colour measurement values after colouring** |
|---|---|---|---|---|
| - | Colour shade of the hair before the colouring treatment | --- | --- | L= 80.60 |
| | | | | C= 12.10 |
| | | | | h= 92.10 |
| **13** | 4-(4-{[methyl(phenyl)hydrazono]-methyl}-1-pyridiniumyl)-1-butanesulfonate (1a) | 9.7 | Bright | L= 76.65 |
| | | | golden | C= 90.42 |
| | | | yellow | h= 93.20 |

### Example 14: Hair colourant with anionic surface-active substances

| | |
|---|---|
| 0.87 g | 4-(4-{[methyl(phenyl)hydrazono]methyl}-1-pyridiniumyl)-1-butanesulfonate (1a) |
| 5.00 g | Ethanol |
| 7.50 g | Lauryl ether sulfate, 28% in water |
| ad 100.00 g | Water, demineralized |

The pH is adjusted to the pH values given in table 4 using 25% ammonia.

The hair colouring is carried out by applying an amount of the colourant sufficient for the hair colouring to the hair and distributing it evenly using a brush. After a contact time of 30 minutes at 40 °C, the hair is rinsed with lukewarm water, washed with a shampoo, rinsed again with lukewarm water and then dried.

The colouring results are summarized in table 4 below.

**Table 4:**

| **Ex.** | **Compound of the formula (I)** | **pH of the colourant** | **Colour shade after colouring** | **Colour measurement values after colouring** |
|---|---|---|---|---|
| **-** | Colour shade of the hair before the colouring treatment | --- | --- | L= 80.60 |
| | | | | C= 12.10 |
| | | | | h= 92.10 |
| **14** | 4-(4- {[methyl(phenyl)hydrazono]-methyl}-1-pyridiniumyl)-1-butanesulfonate (1a) | 9.1 | Bright | L= 80.67 |
| | | | lemon | C= 79.86 |
| | | | yellow | h= 96.70 |

### Examples 15 to 23: Hair colourant with oxidizing agent

| | |
|---|---|
| 0.6 g | Dye of the formula (I), (II) or (III) as in table 5 |
| 5.0 g | Ethanol |
| 4.0 g | Decyl glucoside |
| 0.2 g | Ethylenediaminotetraacetic acid disodium salt |
| ad 100.0 g | Water, demineralized |

5 g of the above colour carrier mass are mixed with 5 g of a 9% strength hydrogen peroxide solution. The pH is adjusted to 9.5 using 25% strength ammonia.

The resulting ready-to-use hair colourant is applied to the hair (yak hair) and distributed evenly using a brush. After a contact time of 30 minutes at 40 °C, the hair is rinsed with lukewarm water, washed with a shampoo, rinsed again with lukewarm water and then dried.

The colouring results are summarized in table 5 below.

**Table 5:**

| **Ex.** | **Compound of the formula (I), (II) or (III) (as in examples 1 to 2)** | **Colour shade after colouring** | **Colour measurement values after colouring** |
|---|---|---|---|
| **-** | Colour shade of the hair before the colouring treatment | --- | L= 80.60 |
| | | | C= 12.10 |
| | | | h= 92.10 |
| **15** | 4-(4-{[methyl(phenyl)hydrazono]-methyl}-1-pyridiniumyl)-1-butanesulfonate (1a) | bright golden | L= 80.52 |
| | | yellow | C= 88.40 |
| | | | h= 94.40 |
| **16** | 4-(3-{[methyl(phenyl)hydrazono]-methyl}-1-pyridiniumyl)-1-butanesulfonate (1b) | Bright lemon | L= 82.38 |
| | | yellow | C= 52.23 |
| | | | h= 99.70 |
| **17** | 4-(2-{[methyl(phenyl)hydrazono]-methyl}-1-pyridiniumyl)-1-butanesulfonate (1c) | Bright lemon | L= 82.75 |
| | | yellow | C= 72.56 |
| | | | h= 98.80 |
| **18** | 4-[4-({[4-(methoxy)phenyl]-hydrazono}methyl)-1-pyridiniumyl]-1-butanesulfonate (1d) | Bright orange | L= 64.13 |
| | | | C= 80.12 |
| | | | h= 65.00 |
| **19** | 4-[4-({methyl[4-(methoxy)-phenyl]hydrazono}methyl)-1-pyridiniumyl]-1-butanesulfonate (1e) | Bright orange- | L= 68.19 |
| | | yellow | C= 81.42 |
| | | | h= 76.30 |
| **20** | 4-(2-{[methyl(phenyl)hydrazono]-methyl}-1,3-thiazol-3-ium-3-yl)-1-butanesulfonate (1f) | Bright lemon | L= 82.24 |
| | | yellow | C= 75.95 |
| | | | h= 97.90 |
| **21** | 4-(4-{[2-(3-methyl-1,3-benzothiazol-2(3*H*)- ylidene)-hydrazono]methyl}-1-pyridiniumyl)-1-butanesulfonate (2a) | orange | L= 73.29 |
| | | | C= 68.90 |
| | | | h= 76.30 |
| | | | |
| **22** | 4-(3-{[2-(3-methyl-1,3-benzothiazol-2(3*H*)-ylidene)-hydrazono]methyl}-1-pyridiniumyl)-1-butanesulfonate (2b) | Lemon yellow | L= 81.29 |
| | | | C= 63.29 |
| | | | h= 96.9 |
| | | | |
| **23** | 4-(4-{[2-(3,4-dimethyl-1,3-thiazol-2(3*H*)-ylidene)hydrazono]methyl}-1-pyridiniumyl)-1-butanesulfonate (2d) | red | L= 51.70 |
| | | | C= 59.96 |
| | | | h= 39.7 |

### Examples 24 to 28: Hair colourant with oxidizing agent

| | |
|---|---|
| 5.0 mmol | Dye of the formula (I), (II) or (III) as in table 6 |
| 5.0 g | Ethanol |
| 4.0 g | Decyl glucoside |
| 0.2 g | Ethylenediaminotetraacetic acid disodium salt |
| ad 100.0 g | Water, demineralized |

5 g of the above colour carrier mass are mixed with 5 g of a 9% strength hydrogen peroxide solution. The pH is adjusted to 9.0 using 25% ammonia.

The resulting ready-to-use hair colourant is applied to natural hair and distributed evenly using a brush. After a contact time of 30 minutes at 40 °C, the hair is rinsed with lukewarm water, washed with a shampoo, rinsed again with lukewarm water and then dried. The washing process is repeated five times. The colours do not change visually.

**Table 6:**

| **Ex.** | **Compound of the formula (I), (II) or (III) (as in examples 1 to 2)** | **Colour shade after colouring** | **Colour shade after washing** |
|---|---|---|---|
| **-** | Colour shade of the hair before the colouring treatment | L=32.07 | |
| | | C=13.40 | |
| | | h= 65.10 | |
| **24** | 4-(4-{[methyl(phenyl)hydrazono]-methyl}-1-pyridiniumyl)-1-butanesulfonate (1a) | middle blond | middle blond with |
| | | with yellow | yellow reflex |
| | | reflex | L= 37.22 |
| | | L= 37.06 | C= 26.40 |
| | | C= 28.27 | h= 77.80 |
| | | h= 78.80 | ΔL= 0.16 |
| **25** | 4-(3-{[methyl(phenyl)hydrazono]-methyl}-1-pyridiniumyl)-1-butanesulfonate (1b) | middle blond | middle blond with |
| | | with yellow | yellow reflex |
| | | reflex | L= 35.09 |
| | | L= 36.92 | C= 20.40 |
| | | C= 22.47 | h= 73.20 |
| | | h= 76.70 | ΔL= 1.83 |
| **26** | 4-(2-{[methyl(phenyl)hydrazono]-methyl}-1,3-thiazol-3-ium-3-yl)-1-butanesulfonate (1f) | middle blond | middle blond with |
| | | with yellow | yellow reflex |
| | | reflex | L= 36.54 |
| | | L= 37.20 | C= 22.57 |
| | | C= 24.43 | h= 75.40 |
| | | h= 77.70 | ΔL = 0.66 |
| **27** | 4-(3-{[2-(3-methyl-1,3-benzothiazol-2(3*H*)-ylidene)- | middle blond | middle blond with |
| | | with yellow | yellow reflex |
| | hydrazono]methyl}-1-pyridiniumyl)-1-butanesulfonate (2b) | reflex | L= 35.20 |
| | | L= 35.09 | C= 18.28 |
| | | C= 18.79 | h= 71.30 |
| | | h= 72.40 | ΔL= 0.11 |

The L*C*h* colour measurement values given in the present examples were ascertained using a colourimeter from Minolta, model Chromameter II. Here, the L value is the lightness (i.e. the lower the L value, the greater the colour intensity), while the C value is a measure of the colourfulness ("chroma") (i.e. the greater the C value, the more colourful the colour). The h value is the colour shade angle ("hue").

Unless stated otherwise, all of the percentages given in the present application are percentages by weight.

## Claims

1. An agent for the nonoxidative colouring of keratin fibres, **characterized by** comprising at least one zwitterionic azomethine dye of the general formula (I), (II) or (III) 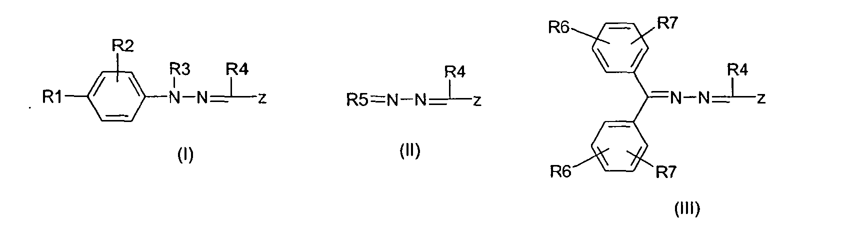 in which
**R1** and **R2** may be identical or different and indepedently of one another, are hydrogen, a halogen atom, a saturated or unsaturated (C₁-C₁₂)-alkyl group, a (C₁-C₁₂)-alkyl group substituted by a halogen atom, a hydroxyl group, a hydroxy-(C₁-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted C(O)O-phenyl group or an unsubstituted C(O)O-phenyl group, a substituted phenyl group or an unsubstituted phenyl group, a substituted naphthyl group or an unsubstituted naphthyl group, a substituted heteroaryl group or an unsubstituted heteroaryl group;
**R3** is an hydrogen, a saturated or unsaturated (C₁-C₁₂)-alkyl group, a hydroxy-(C₂-C₁₂)alkyl group, or forms with a carbon atom of the benzene ring a five or six-membered heterocycle which may be substituted with one or more (C₁-C₁₂)-alkyl group;
**R4** is hydrogen, a saturated or unsaturated (C₁-C₁₂)-alkyl group, a substituted phenyl group or an unsubstituted phenyl group;
**R5** is chosen from a group according to the general formula 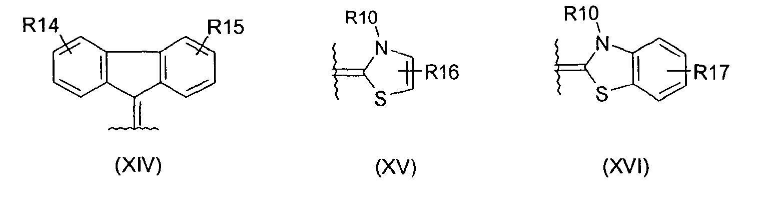 **R6** and **R7** may be identical or different and, independently of one another, are hydrogen, a halogen atom, an amino group, a (C₁-C₁₂)-alkylamino group, a (C₁-C₁₂)-dialkylamino group, a C₁-C₆-alkylcyano group, a hydroxyl group, a nitro group, a saturated or unsaturated (C₁-C₁₂)-alkyl group, a hydroxy-(C₁-C₁₂)-alkyl group, a (C₁-C₁₂)-alkoxy group, a methoxy-(C₁-C₁₂)-alkyl group, a C(O)O-(C₁-C₁₂)-alkyl group;
z is a heterocycle of the formula (IV) to (XII) 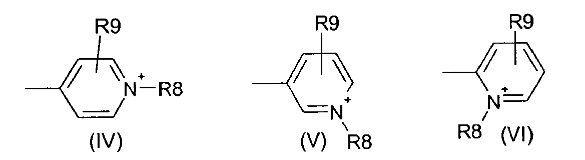 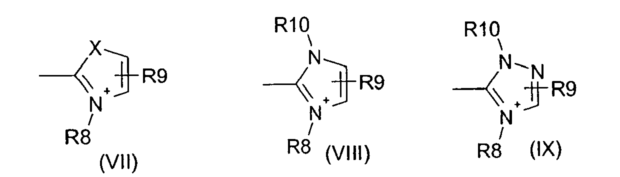 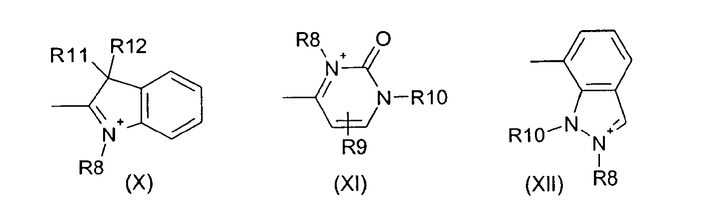 X is oxygen or sulfur;
**R8** is an alkyl sulfonate radical of the formula (XIII); 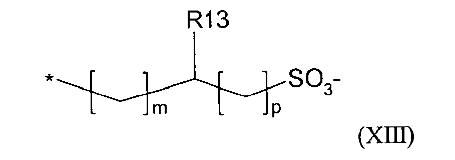 **R9** is hydrogen, a halogen atom, a saturated or unsaturated (C₁-C₁₂)-alkyl group, a (C₁-C₁₂)-alkyl group substituted by a halogen atom, a hydroxyl group, a hydroxy-(C₂-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted or unsubstituted C(O)O-phenyl group, a substituted or unsubstituted phenyl group, or a benzene ring condensed to the heteroaromatic ring of formula (IV) to (XII);
**R10** is a saturated or unsaturated (C₁-C₁₂)-alkyl group or a saturated or unsaturated hydroxy-(C₁-C₁₂)-alkyl group;
**R11** and **R12** may be identical or different and indepedently of one another, are a saturated or unsaturated (C₁-C₁₂)-alkyl group;
**R13** is hydrogen or a hydroxyl group;
**R14, R15, R16** and **R17** may be identical or different and indepedently of one another, are hydrogen, a halogen atom, a saturated or unsaturated (C₁-C₁₂)-alkyl group, a (C₁-C₁₂)-alkyl group substituted by a halogen atom, a hydroxyl group, a hydroxy-(C₁-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted C(O)O-phenyl group or an unsubstituted C(O)O-phenyl group, a substituted phenyl group or an unsubstituted phenyl group,
**m** is equal to 0 to (n-1);
**p** is equal to 0 to (n-1) with m+p = (n-1) and
**n** is an integer from 1 to 6.

2. An agent for the simultaneous lightening and colouring of keratin fibres, **characterized by** comprising at least one zwitterionic azomethine dye of the general formula (I), (II) or (III) 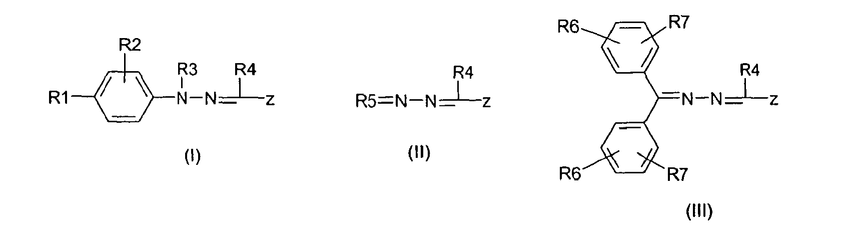 in which
**R1** and **R2** may be identical or different and indepedently of one another, are hydrogen, a halogen atom, a saturated or unsaturated (C₁-C₁₂)-alkyl group, a (C₁-C₁₂)-alkyl group substituted by a halogen atom, a hydroxyl group, a hydroxy-(C₁-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted C(O)O-phenyl group or an unsubstituted C(O)O-phenyl group, a substituted phenyl group or an unsubstituted phenyl group, a substituted naphthyl group or an unsubstituted naphthyl group, a substituted heteroaryl group or an unsubstituted heteroaryl group;
R3 is an hydrogen, a saturated or unsaturated (C₁-C₁₂)-alkyl group, a hydroxy-(C₂-C₁₂)alkyl group, or forms with a carbon atom of the benzene ring a five or six-membered heterocycle which may be substituted with one or more (C₁-C₁₂)-alkyl group;
**R4** is hydrogen, a saturated or unsaturated (C₁-C₁₂)-alkyl group, a substituted phenyl group or an unsubstituted phenyl group;
**R5** is chosen from a group according to the general formula 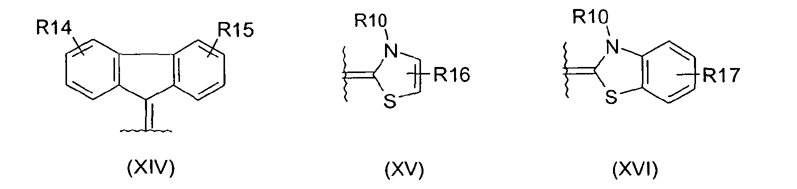 **R6** and **R7** may be identical or different and, independently of one another, are hydrogen, a halogen atom, an amino group, a (C₁-C₁₂-alkylamino group, a (C₁-C₁₂)-dialkylamino group, a C₁-C₆-alkylcyano group, a hydroxyl group, a nitro group, a saturated or unsaturated (C₁-C₁₂)-alkyl group, a hydroxy-(C₁-C₁₂)-alkyl group, a (C₁-C₁₂)-alkoxy group, a methoxy-(C₁-C₁₂)-alkyl group, a C(O)O-(C₁-C₁₂)-alkyl group;
**z** is a heterocycle of the formula (IV) to (XII) 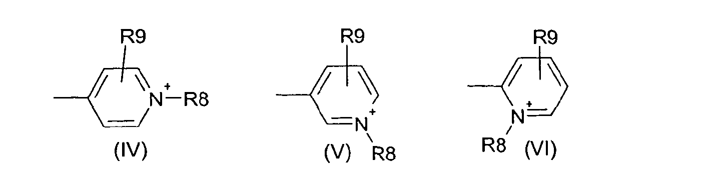 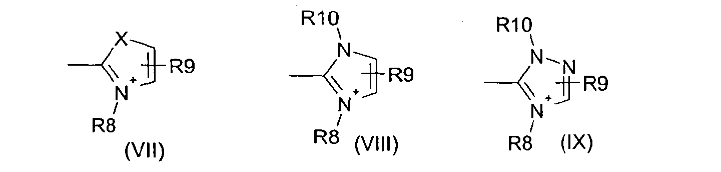 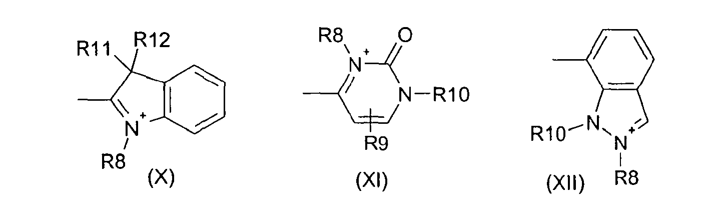 X is oxygen or sulfur;
**R8** is an alkyl sulfonate radical of the formula (XIII); 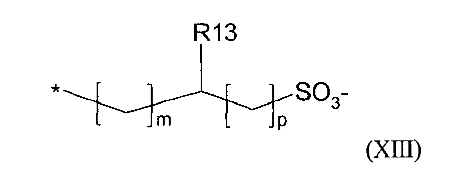 **R9** is hydrogen, a halogen atom, a saturated or unsaturated (C₁-C₁₂)-alkyl group, a (C₁-C₁₂)-alkyl group substituted by a halogen atom, a hydroxyl group, a hydroxy-(C₂-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted or unsubstituted C(O)O-phenyl group, a substituted or unsubstituted phenyl group, or a benzene ring condensed to the heteroaromatic ring of formula (IV) to (XII);
**R10** is a saturated or unsaturated (C₁-C₁₂)-alkyl group or a saturated or unsaturated hydroxy-(C₁-C₁₂)-alkyl group;
**R11** and **R12** may be identical or different and indepedently of one another, are a saturated or unsaturated (C₁-C₁₂)-alkyl group;
R13 is hydrogen or a hydroxyl group;
**R14, R15, R16** and **R17** may be identical or different and indepedently of one another, are hydrogen, a halogen atom, a saturated or unsaturated (C₁-C₁₂)-alkyl group, a (C₁-C₁₂)-alkyl group substituted by a halogen atom, a hydroxyl group, a hydroxy-(C₁-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted C(O)O-phenyl group or an unsubstituted C(O)O-phenyl group, a substituted phenyl group or an unsubstituted phenyl group,
**m** is equal to 0 to (n-1);
**p** is equal to 0 to (n-1) with m+p = (n-1) and
**n** is an integer from 1 to 6.

3. An oxidative colourant for colouring fibres based on at least one oxidation dye precursor, **characterized by** comprising at least one zwitterionic azomethine dye of the general formula (I), (II) or (III) 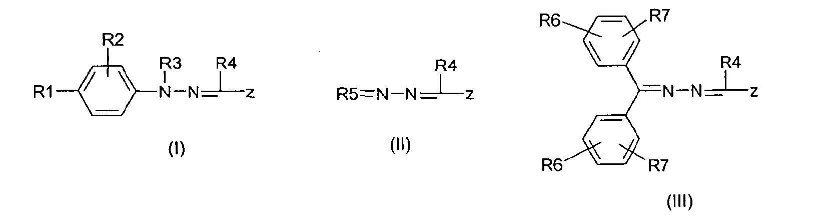 in which
**R1** and **R2** may be identical or different and indepedently of one another, are hydrogen, a halogen atom, a saturated or unsaturated (C₁-C₁₂)-alkyl group, a (C₁-C₁₂)-alkyl group substituted by a halogen atom, a hydroxyl group, a hydroxy-(C₁-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted C(O)O-phenyl group or an unsubstituted C(O)O-phenyl group, a substituted phenyl group or an unsubstituted phenyl group, a substituted naphthyl group or an unsubstituted naphthyl group, a substituted heteroaryl group or an unsubstituted heteroaryl group;
**R3** is an hydrogen, a saturated or unsaturated (C₁-C₁₂)-alkyl group, a hydroxy-(C₂-C₁₂)alkyl group, or forms with a carbon atom of the benzene ring a five or six-membered heterocycle which may be substituted with one or more (C₁-C₁₂)-alkyl group;
**R4** is hydrogen, a saturated or unsaturated (C₁-C₁₂)-alkyl group, a substituted phenyl group or an unsubstituted phenyl group;
**R5** is chosen from a group according to the general formula 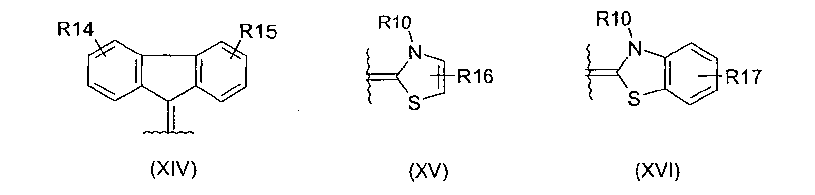 **R6** and **R7** may be identical or different and, independently of one another, are hydrogen, a halogen atom, an amino group, a (C₁-C₁₂)-alkylamino group, a (C₁-C₁₂)-dialkylamino group, a C₁-C₆-alkylcyano group, a hydroxyl group, a nitro group, a saturated or unsaturated (C₁-C₁₂)-alkyl group, a hydroxy-(C₁-C₁₂)-alkyl group, a (C₁-C₁₂)-alkoxy group, a methoxy-(C₁-C₁₂)-alkyl group, a C(O)O-(C₁-C₁₂)-alkyl group;
z is a heterocycle of the formula (IV) to (XII) 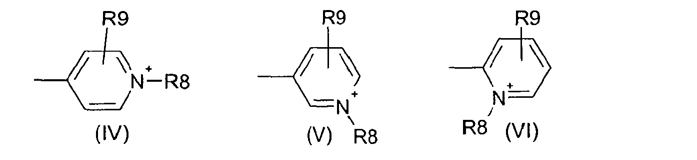 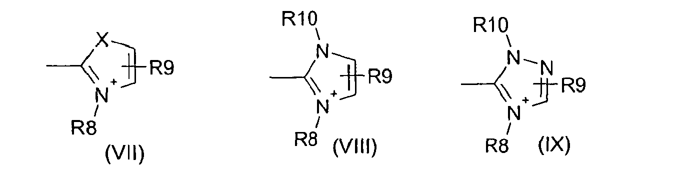 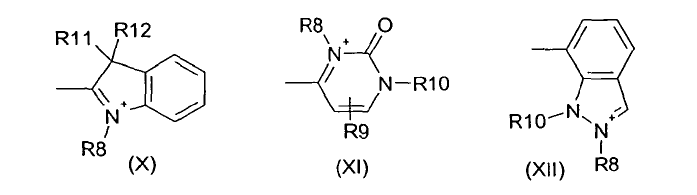 X is oxygen or sulfur;
**R8** is an alkyl sulfonate radical of the formula (XIII); 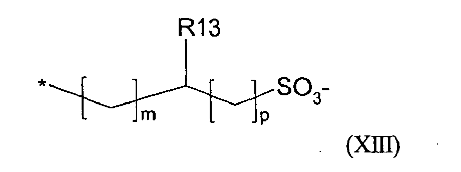 **R9** is hydrogen, a halogen atom, a saturated or unsaturated (C₁-C₁₂)-alkyl group, a (C₁-C₁₂)-alkyl group substituted by a halogen atom, a hydroxyl group, a hydroxy-(C₂-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted or unsubstituted C(O)O-phenyl group, a substituted or unsubstituted phenyl group, or a benzene ring condensed to the heteroaromatic ring of formula (IV) to (XII);
**R10** is a saturated or unsaturated (C₁-C₁₂)-alkyl group or a saturated or unsaturated hydroxy-(C₁-C₁₂)-alkyl group;
**R11** and **R12** may be identical or different and indepedently of one another, are a saturated or unsaturated (C₁-C₁₂)-alkyl group;
**R13** is hydrogen or a hydroxyl group;
**R14, R15, R16** and **R17** may be identical or different and indepedently of one another, are hydrogen, a halogen atom, a saturated or unsaturated (C₁-C₁₂)-alkyl group, a (C₁-C₁₂)-alkyl group substituted by a halogen atom, a hydroxyl group, a hydroxy-(C₁-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid group, a C(O)O-(C₁-C₁₂)-alkyl group, a substituted C(O)O-phenyl group or an unsubstituted C(O)O-phenyl group, a substituted phenyl group or an unsubstituted phenyl group,
**m** is equal to 0 to (n-1);
**p** is equal to 0 to (n-1) with m+p = (n-1) and
**n** is an integer from 1 to 6.

4. The agent as claimed in claim 1, **characterized by** comprising at least one polymer selected from the group consisting of natural polymers customary for cosmetic agents, synthetic polymers or modified polymers of natural origin, and being in the form of a colour setting composition.

5. The agent as claimed in claim 2, **characterized by** the fact that the oxidizing agent is chosen from hydrogen peroxide or its addition compounds onto urea, melamine, sodium borate or sodium carbonate.

6. The agent as claimed in claim 3, **characterized by** the fact that the oxidizing agent is chosen from hydrogen peroxide or its addition compounds onto urea, melamine, sodium borate or sodium carbonate, and persulphates.

7. The agent as claimed in one of claims 1 to 6, **characterized by** the fact that in the formula (I), (II) and (III) **R13** is hydrogen and **n** is 2 or 3.

8. The agent as claimed in one of claims 1 to 7, **characterized by** the fact that the zwitterionic azomethine dye of the formula (I), (II) or (III) is selected from the group consisting of 4-(4-{[methyl(phenyl)hydrazono]methyl}-1-pyridiniumyl)-1-butanesulfonate,
4 (3{[methyl(phenyl)hydrazono]methyl}-1-pyridiniumyl)-1-butanesulfonate,
4-(2-{[methyl(phenyl)hydrazono]methyl}-1-pyridiniumyl)-1-butanesulfonate,
4-(4-{[(4-methoxyphenyl)(methyl)hydrazono]methyl}-1-pyridiniumyl)-1-butanesulfonate,
4-(3-{[(4-methoxyphenyl)(methyl)hydrazono]methyl}-1-pyridiniumyl)-1-butanesulfonate,
4-(2-{[(4-methoxyphenyl)(methyl)hydrazono]methyl}-1-pyridiniumyl)-1-butanesulfonate,
4-(4-{[(2-methoxyphenyl)(methyl)hydrazono]methyl}-1-pyridiniumyl)-1-butanesulfonate,
4-(3-{[(2-methoxyphenyl)(methyl)hydrazono]methyl}-1-pyridiniumyl)-1-butanesulfonate,
4-(2-{[(2-methoxyphenyl)(methyl)hydrazono]methyl}-1-pyridiniumyl)-1-butanesulfonate,
4-(4-{[(4-hydroxyphenyl)(methyl)hydrazono]methyl}-1-pyridiniumyl)-1-butanesulfonate,
4-(3-{[(4-hydroxyphenyl)(methyl)hydrazono]methyl}-1-pyridiniumyl)-1-butanesulfonate,
4-(2-{[(4-hydroxyphenyl)(methyl)hydrazono]methyl}-1-pyridiniumyl)-1-butanesulfonate,
4-(4-{[methyl(4-methylphenyl)hydrazono]methyl}-1-pyridiniumyl)-1-butanesulfonate,
4-(3-{[methyl(4-methylphenyl)hydrazono]methyl}-1-pyridiniumyl)-1-butanesulfonate,
4-(2-{[methyl(4-methylphenyl)hydrazono]methyl}-1-pyridiniumyl)-1-butanesulfonate,
4-(4-{[(4-chlorophenyl)(methyl)hydrazono]methyl}-1-pyridiniumyl)-1-butanesulfonate,
4-(3-{[(4-chlorophenyl)(methyl)hydrazono]methyl}-1-pyridiniumyl)-1-butanesulfonate,
4-(2-{[(4-chlorophenyl)(methyl)hydrazono]methyl}-1-pyridiniumyl)-1-butanesulfonate,
4-(4-{[methyl(4-nitrophenyl)hydrazono]methyl}1-pyridiniumyl)-1-butanesulfonate,
4-(3-{[methyl(4-nitrophenyl)hydrazono]methyl}-1-pyridiniumyl)-1-butanesulfonate,
4-(2-{[methyl(4-nitrophenyl)hydrazono]methyl}-1-pyridiniumyl)-1-butanesulfonate,
4-{4-[(phenylhydrazono)methyl]-1-pyridiniumyl}-1-butanesulfonate,
4-{3-[(phenylhydrazono)methyl]-1-pyridiniumyl}-1-butanesulfonate,
4-{2-[(phenylhydrazono)methyl]-1-pyridiniumyl}-1-butanesulfonate,
4-(4-{[(4-methoxyphenyl)hydrazono]methyl}-1-pyridiniumyl)-1-butanesulfonate,
4-(3-{[(4-methoxyphenyl)hydrazono]methyl}-1-pyridiniumyl)-1-butanesulfonate,
4-(2-{[(4-methoxyphenyl)hydrazono]methyl}-1-pyridiniumyl)-1-butanesulfonate,
4-{4-[*N*-methyl-*N*-phenylethanehydrazonoyl]-1-pyridiniumyl}-1-butanesulfonate,
4-{3-[*N*-methyl-*N*-phenylethanehydrazonoyl]-1-pyridiniumyl}-1-butanesulfonate,
4-{2-[*N*-methyl-*N*-phenylethanehydrazonoyl]-1-pyridiniumyl}-1-butanesulfonate,
4-{4-[*N*-phenylethanehydrazonoyl]-1-pyridiniumyl}-1-butanesulfonate,
4-{3-[*N*-phenylethanehydrazonoyl]-1-pyridiniumyl}-1-butanesulfonate,
4-{2[*N*-phenylethanehydrazonyl]-1-pyridiniumyl}-1-butanesulfonate,
4-(2-{[methyl(phenyl)hydrazono]methyl}-1,3-thiazol-3-ium-3-yl)-1-butanesulfonate,
4-(1-methyl-2-{[methyl(phenyl)hydrazono]methyl}-1H-imidazol-3-ium-3-yl)-1-butanesulfonate,
4-(2-{[methyl(phenyl)hydrazono]methyl}-1,3-oxazol-3-ium-3-yl)-1-butanesulfonate,
4-(4-methyl-5-{[methyl(phenyl)hydrazono]methyl}-4*H*-1,2,4-triazol-1-ium-1-yl)-1-butanesulfonate,
4-(4-{[methyl(phenyl)hydrazono]methyl}-1-quinoliniumyl)-1-butanesulfonate,
4-(2-{[methyl(phenyl)hydrazono]methyl}-1-quinoliniumyl)-1-butanesulfonate,
4-{4-[(2,3-dihydro-1*H*-indol-1-ylimino)methyl]-1-pyridiniumyl}-1-butanesulfonate,
4-(4-{[(2,2,3,3-tetramethyl-2,3-dihydro-1*H*-indol-1-yl)imino]methyl}-1-pyridiniumyl)-1-butanesulfonate,
4-(3,3-dimethyl-2-{[methyl(phenyl)hydrazono]methyl}-3*H*-indolium-1-yl)-1-butanesulfonate,
4-(2-{[(4-methoxyphenyl)(methyl)hydrazono]methyl}-3,3-dimethyl-3*H*-indolium-1-yl)-1-butanesulfonate,
4-(3-methyl-6-{[methyl(phenyl)hydrazono]methyl}-2-oxo-2,3-dihydropyrimidin-1-ium-1-yl)-1-butanesulfonate,
4-(1-methyl-7-{[methyl(phenyl)hydrazono]methyl}-1*H*-indazol-2-ium-2-yl)-1-butanesulfonate,
3-(4-{[methyl(phenyl)hydrazono]methyl}-1-pyridiniumyl)-1-propanesulfonate,
3-(3-{[methyl(phenyl)hydrazono]methyl}-1-pyridiniumyl)-1-propanesulfonate,
3-(2-{[methyl(phenyl)hydrazono]methyl}-1-pyridiniumyl)-1-propanesulfonate,
3-(4-{[(4-methoxyphenyl)(methyl)hydrazono]methyl}-1-pyridiniumyl)-1-propanesulfonate,
3-(3-{[(4-methoxyphenyl)(methyl)hydrazono]methyl}-1-pyridiniumyl)-1-propanesulfonate,
3-(2-{[(4-methoxyphenyl)(methyl)hydrazono]methyl}-1-pyridiniumyl)-1-propanesulfonate,
3-(4-{[(2-methoxyphenyl)(methyl)hydrazono]methyl}1-pyridiniumyl)-1-propanesulfonate,
3-(3-{[(2-methoxyphenyl)(methyl)hydrazono]methyl}-1-pyridiniumyl)-1-propanesulfonate,
3-(2-{[(2-methoxyphenyl)(methyl)hydrazono]methyl}-1-pyridiniumyl)-1-propanesulfonate,
3-(4-{[(4-hydroxyphenyl)(methyl)hydrazono]methyl}-1-pyridiniumyl)-1-propanesulfonate,
3-(3-{[(4-hydroxyphenyl)(methyl)hydrazono]methyl}-1-pyridiniumyl)-1-propanesulfonate,
3-(2-{[(4-hydroxyphenyl)(methyl)hydrazono]methyl}-1-pyridiniumyl)-1-propanesulfonate,
3-(4-{[methyl(4-methylphenyl)hydrazono]methyl}-1-pyridiniumyl)-1-propanesulfonate,
3-(3-{[methyl(4-methytphenyl)hydrazono]methyl}-1-pyridiniumyl)-1-propanesulfonate,
3-(2-{[methyl(4-methylphenyl)hydrazono]methyl}-1-pyridiniumyl)-1-propanesulfonate,
3-(4-{[(4-chlorophenyl)(methyl)hydrazono]methyl}-1-pyridiniumyl)-1- propanesulfonate,
3-(3-{[(4-chlorophenyl)(methyl)hydrazono]methyl}-1-pyridiniumyl)-1- propanesulfonate,
3-(2-{[(4-chlorophenyl)(methyl)hydrazono]methyl}-1-pyridiniumyl)-1- propanesulfonate,
3-(4-{[methyl(4-nitrophenyl)hydrazono]methyl}-1-pyridiniumyl)-1-propanesulfonate,
3-(3-{[methyl(4-nitrophenyl)hydrazono]methyl}-1-pyridiniumyl)-1-propanesulfonate,
3-(2-{[methyl(4-nitrophenyl)hydrazono]methyl}-1-pyridiniumyl)-1-propanesulfonate,
3-{4-[(phenylhydrazono)methyl]-1-pyridiniumyl}-1-propanesulfonate,
3-{3-[(phenylhydrazono)methyl]-1-pyridiniumyl}-1-propanesulfonate,
3-{2-[(phenylhydrazono)methyl]-1-pyridiniumyl}-1-propanesulfonate,
3-(4-{[(4-methoxyphenyl)hydrazono]methyl}-1-pyridiniumyl)-1-propanesulfonate,
3-(3-{[(4-methoxyphenyl)hydrazono]methyl}-1-pyridiniumyl)-1-propanesulfonate,
3-(2-{[(4-methoxyphenyl)hydrazono]methyl}-1-pyridiniumyl)-1- propanesulfonate,
3-{4-[*N*-methyl-*N*-phenylethanehydrazonoyl]-1-pyridiniumyl}-1-propanesulfonate,
3-{3-[*N*-methyl-*N*-phenylethanehydrazonoyl]-1-pyridiniumyl}-1-propanesulfonate,
3-{2-[*N*-methyl-*N*-phenylethanehydrazonoyl]-1-pyridiniumyl}-1-propanesulfonate,
3-{4-[*N*-phenylethanehydrazonoyl]-1-pyridiniumyl}-1-propanesulfonate,
3-{3-[*N*-phenylethanehydrazonoyl]-1-pyridiniumyl}-1-propanesulfonate,
3-{2-[*N*-phenylethanehydrazonoyl]-1-pyridiniumyl}-1-propanesulfonate,
3-(2-{[methyl(phenyl)hydrazono]methyl}-1,3-thiazol-3-ium-3-yl)-1- propanesulfonate,
3-(1-methyl-2-{[methyl(phenyl)hydrazono]methyl}-1H-imidazol-3-ium-3-yl)-1-propanesulfonate,
3-(2-{[methyl(phenyl)hydrazono]methyl}-1,3-oxazol-3-ium-3-yl)-1-propanesulfonate,
3-(4-methyl-5-{[methyl(phenyl)hydrazono]methyl}-4*H*-1,2,4-triazol-1-ium-1-yl)-1-propanesulfonate,
3-(4-{[methyl(phenyl)hydrazono]methyl}-1-quinoliniumyl)-1-propanesulfonate,
3-(2-{[methyl(phenyl)hydrazono]methyl}-1-quinoliniumyl)-1-propanesulfonate,
3-{4-[(2,3-dihydro-1*H*-indol-1-ylimino)methyl]-1-pyridiniumyl}-1-propanesulfonate
3-(4-{[(2,2,3,3-tetramethyl-2,3-dihydro-1*H*-indol-1-yl)imino]methyl}-1-pyridiniumyl)-1-propanesulfonate,
3-(3,3-dimethyl-2-{[methyl(phenyl)hydrazono]methyl}-3*H*-indolium-1-yl)-1-propanesulfonate,
3-(2-{[(4-methoxyphenyl)(methyl)hydrazono]methyl}-3,3-dimethyl-3*H*-indolium-1-yl)-1-propanesulfonate,
3-(3-methyl-6- {[methyl(phenyl)hydrazono]methyl}-2-oxo-2,3-dihydropyrimidin-1-ium-1-yl)-1-propanesulfonate,
3-(1-methyl-7-{[methyl(phenyl)hydrazono]methyl}-1*H*-indazol-2-ium-2-yl)-1-propanesulfonate,
4-(4-{[(3-methyl-1,3-benzothiazol-2(3H)-ylidene)hydrazono]methyl}-1-pyridiniumyl)-1-butanesulfonate,
4-(3-{[(3-methyl-1,3-benzothiazol-2(3H)-ylidene)hydrazono]methyl}-1-pyridiniumyl)-1-butanesulfonate,
4-(4-{[(3,4-dimethyl-1,3-thiazol-2(3H)-ylidene)hydrazono]methyl}-1-pyridiniumyl)-1-butanesulfonate,
4-(3-{[(3,4-dimethyl-1,3-thiazol-2(3H)-ylidene)hydrazono]methyl}-1-pyridiniumyl)-1-butanesulfonate,
4-(4-{[(3,4,5-trimethyl-1,3-thiazol-2(3H)-ylidene)hydrazono]methyl}-1-pyridiniumyl)-1-butanesulfonate,
4-(3-{[(3,4,5-trimethyl-1,3-thiazol-2(3H)-ylidene)hydrazono]methyl}-1-pyridiniumyl)-1-butanesulfonate,
4-(1-methyl-2-{[2-(3-methyl-1,3-benzothiazol-2(3*H*)-ylidene)hydrazono]methyl}-1*H-*imidazol-3-ium-3-yl)-1-butanesulfonate,
4-(2-{[2-(3,4-dimethyl-1,3-thiazol-2(3*H*)-ylidene)hydrazono]methyl}-1-methyl-1*H-*imidazol-3-ium-3-yl)-1-butanesulfonate,
4-(2-{[2-(3,4,5-trimethyl-1,3-thiazol-2(3*H*)-ylidene)hydrazono]methyl}-1-methyl-1*H-*imidazol-3-ium-3-yl)-1-butanesulfonate,
4-{4-[({bis[4-(dimethylamino)phenyl]methylene}hydrazono)methyl]-1-pyridiniumyl}-1-butanesulfonate,
4-{3-[({bis[4-(dimethylamino)phenyl]methylene}hydrazono)methyl]-1-pyridiniumyl}-1-butanesulfonate,
4-{4-[(9*H*-fluoren-9-ylidenehydrazono)methyl]-1-pyridiniumyl}-1-butanesulfonate,
4-{4-[({bis[4-(dimethylamino)phenyl]methylene}hydrazono)methyl]-1-quinoliniumyl}-1-butanesulfonate,
3-(4-{[(3-methyl-1,3-benzothiazol-2(3H)-ylidene)hydrazono]methyl}-1-pyridiniumyl)-1-propanesulfonate,
3-(3-{[(3-methyl-1,3-benzothiazol-2(3H)-ylidene)hydrazono]methyl}-1-pyridiniumyl)-1-propanesulfonate,
3-(4-{[(3,4-dimethyl-1,3-thiazol-2(3H)-ylidene)hydrazono]methyl}-1-pyridiniumyl)-1-propanesulfonate,
3-(3-{[(3,4-dimethyl-1,3-thiazol-2(3H)-ylidene)hydrazono]methyl}-1-pyridiniumyl)-1-propanesulfonate,
3-(4-{[(3,4,5-trimethyl-1,3-thiazol-2(3H)-ylidene)hydrazono]methyl}-1-pyridiniumyl)-1-propanesulfonate,
3-(3-{[(3,4,5-trimethyl-1,3-thiazol-2(3H)-ylidene)hydrazono]methyl}-1-pyridiniumyl)-1-propanesulfonate,
3-(1-methyl-2-{[2-(3-methyl-1,3-benzothiazol-2(3*H*)-ylidene)hydrazono]methyl}-1*H-*imidazol-3-ium-3-yl)-1-propanesulfonate,
3-(2-{[2-(3,4-dimethyl-1,3-thiazol-2(3*H*)-ylidene)hydrazono]methyl}-1-methyl-1*H-*imidazol-3-ium-3-yl)-1-propanesulfonate,
3-(2-{[2-(3,4,5-trimethyl-1,3-thiazol-2(3*H*)-ylidene)hydrazono]methyl}-1-methyl-1*H-*imidazol-3-ium-3-yl)-1-propanesulfonate,
3-{4-[({bis[4-(dimethylamino)phenyl]methylene}hydrazono)methyl]-1-pyridiniumyl}-1-propanesulfonate,
3-{3-[({bis[4-(dimethylamino)phenyl]methylene}hydrazono)methyl]-1-pyridiniumyl}-1-propanesulfonate,
3-(4-[(9*H*-fluoren-9-ylidenehydrazono)methyl]-1-pyridiniumyl)-1-propanesulfonate and
3-{4-[({bis[4-(dimethylamino)phenyl]methylene}hydrazono)methyl]-1-quinoliniumyl}-1-propanesulfonate.

9. The agent as claimed in one of claims 1 to 8, **characterized by** the fact that the zwitterionic azomethine dye of the formula (I), (II) or (III) is present in an amount of from 0.01 to 10% by weight.

10. The agent as claimed in one of claims 1 to 10, **characterized by** th fact that it additionally comprises at least one further direct dye which is selected from the group consisting of nitro dyes, azo dyes, anthraquinone dyes, triphenylmethane dyes, basic dyes and acidic dyes.

11. The agent as claimed in claim 11, **characterized by** the fact that the additional direct dye is present in a total amount of from 0.01 to 15% by weight.

12. The agent as claimed in one of claims 1 to 11, **characterized by** the fact that it has a pH-value of from 2 to 11.

13. The agent as claimed in one of claims 1 to 12, **characterized by** th fact that it is a hair colourant.
